# EUROPÄISCHE PATENTSCHRIFT

(11) **EP 3 058 413 B1**
(45) Veröffentlichungstag und Bekanntmachung des Hinweises auf die Patenterteilung: **21.04.2021**
(21) Anmeldenummer: 14753055.4
(22) Anmeldetag: 15.08.2014
(51) Int. Cl.: G02B 21/00, G02B 21/22, A61B 90/20

(54) **OPERATIONSMIKROSKOP MIT OPTISCHEN SCHNITTSTELLEN**
OPERATING MICROSCOPE WITH OPTICAL INTERFACES
MICROSCOPE OPÉRATOIRE MUNI D'INTERFACES OPTIQUES

(30) Priorität: 20.08.2013 DE 102013216476
(43) Veröffentlichungstag der Anmeldung: 24.08.2016
(73) Patentinhaber: Carl Zeiss Meditec AG, 07745 Jena (DE)
(72) Erfinder: MÜLLER, André, 89551 Königsbronn-Zang (DE); KOLSTER, Daniel, 73447 Oberkochen (DE); LÜCKE, Christian, 73447 Oberkochen (DE); REIMER, Peter, 73479 Ellwangen (DE)
(74) Vertreter: Gauss, Nikolai
(86) Internationale Anmeldenummer: PCT/EP2014/067491
(87) Internationale Veröffentlichungsnummer: WO 2015/024877

(56) Entgegenhaltungen:
- EP-A2- 1 235 094
- DE-A1- 3 318 011
- DE-A1-102006 050 846
- DE-U1- 29 923 951
- US-A- 3 173 984
- US-A- 4 685 776
- US-A- 5 579 772
- US-A- 5 867 309
- US-A- 6 081 371
- US-A- 6 088 155
- US-A1- 2001 010 592
- US-B2- 6 804 051
- Dr. N. Gauss, Carl Zeiss AG: "Einspruch (gegen EP1235094)", , 19. Januar 2007 (2007-01-19), XP002736692, Gefunden im Internet: URL:https://register.epo.org/application?d ocumentId=ELHLTNVG9067FI4&number=EP0210016 2&lng=en&npl=false [gefunden am 2015-02-25]

## Beschreibung

Die Erfindung betrifft ein Operationsmikroskop für das stereoskopische Beobachten eines Operationsbereichs mit einem ersten und mit einem zweiten stereoskopischen Teilstrahlengang und mit einem Einkoppelmodul für das wahlweise Einkoppeln eines an einer optischen Schnittstelle bereitgestellten Strahlengangs in den ersten oder zweiten stereoskopischen Teilstrahlengang. Darüber hinaus betrifft die Erfindung ein Operationsmikroskop mit einem Auskoppelmodul für das wahlweise Auskoppeln eines Strahlengangs aus dem ersten oder dem zweiten stereoskopischen Teilstrahlengang in dem Operationsmikroskop zu einer optischen Schnittstelle.

Außerdem betrifft die Erfindung ein Ein- und Auskoppelmodul für das wahlweise Ein- bzw. Auskoppeln eines Strahlengangs in einem optischen Instrument, insbesondere in einem Operationsmikroskop, sowie ein Operationsmikroskop mit einem derartigen Ein- und Auskoppelmodul.

Ein Auskoppelmodul der eingangs genannten Art ist aus der DE 10 206 050 846 A1 bekannt. In der US 6,804,051 B2 ist ein Ein- und Auskoppelmodul beschrieben, mit dem bei einem Operationsmikroskop ein Strahlengang wahlweise von einer ersten optischen Schnittstelle in einen ersten oder in zweiten stereoskopischen Teilstrahlengang des Operationsmikroskop eingekoppelt werden kann. Hier ist es möglich, zusätzlich aus dem ersten oder dem zweiten stereoskopischen Teilstrahlengang einen weiteren Strahlengang zu einer weiteren optischen Schnittstelle auszukoppeln. Das Ein- und Auskoppelmodul enthält hierzu einen in dem ersten stereoskopischen Teilstrahlengang angeordneten ersten Strahlteiler und einen in dem zweiten stereoskopischen Teilstrahlengang angeordneten zweiten Strahlteiler. In dem Ein- und Auskoppelmodul gibt es eine erste einstellbare Optikbaugruppe, die den Strahlengang von der ersten optischen Schnittstelle wahlweise dem ersten Strahlteiler oder dem zweiten Strahlteiler zuführt. Das Ein- und Auskoppelmodul enthält außerdem eine weitere einstellbare Optikbaugruppe, um den aus dem ersten oder dem zweiten stereoskopischen Teilstrahlengang des Operationsmikroskops ausgekoppelten Strahlengang zu der weiteren optischen Schnittstelle zu führen. Die einstellbaren Optikbaugruppen in dem Ein- und Auskoppelmodul enthalten für das Umschalten des an der optischen Schnittstelle für das Einkoppeln bereitgestellten Strahlengangs und des der optischen Schnittstelle für das Auskoppeln zugeführten Strahlengangs jeweils zwei aus einer ersten Position in eine zweite Position und umgekehrt relativ zu den optischen Schnittstellen linearbeweglich verlagerbare Prismenblöcke. Diese Prismenblöcke haben jeweils zwei Spiegelflächen. Von diesen Prismenblöcken ist jeweils einer in dem ein- und ein anderer in dem auszukoppelnden Strahlengang positioniert.

Die EP 1 666 948 A2 beschreibt ein Operationsmikroskop mit einem Einkoppelmodul. In diesem Operationsmikroskop ist zwischen dem ersten (linken) und dem zweiten (rechten) stereoskopischen Teilstrahlengang für das stereoskopische Beobachten eines Objektbereichs durch ein Mikroskop-Hauptobjektiv hindurch ein drehbarer Spiegel angeordnet. Mit diesem drehbaren Spiegel kann die an einer optischen Schnittstelle zugeführte Bildinformation von einem Display wahlweise zu einem in dem linken und rechten Beobachtungsstrahlengang angeordneten Strahlteiler geführt werden. Entsprechend der Position des drehbaren Spiegels wird die an dem Display angezeigte Bildinformation dann entweder in den linken oder den rechten Beobachtungsstrahlengang eingekoppelt und auf diese Weise dem Beobachtungsbild des Objektbereichs überlagert.

In der EP 1 486 813 A1 ist ein Operationsmikroskop mit einem Auskoppelmodul beschrieben, das eine optische Schnittstelle aufweist, an der wahlweise das Licht aus einem linken und rechten stereoskopischen Teilstrahlengang für die Objektbeobachtung bereitgestellt werden kann. In dem ersten und dem zweiten stereoskopischen Teilstrahlengang des Operationsmikroskops gibt es hierfür drehbeweglich gelagerte Strahlteiler, die das Beobachtungslicht zu einem schaltbaren Spiegelelement führen, das es zu einer optischen Schnittstelle lenkt.

Aus der US 3,173,984 ist ein Stereomikroskop mit einem verlagerbaren Prisma bekannt, das in einem linken und einem rechten Teilstrahlengang für das linke und rechte Beobachterauge verlagert werden kann, um einer Kamera entsprechende Bildinformation zuzuführen.

Die DE 33 18 011 A1 offenbart ein Mikroskop mit einer seitlich angeordneten Schnittstelle und mit einem Spiegelprisma, das mittels einer Handhabe in einen stereoskopischen Teil-Strahlengang eingeführt werden kann, um das Licht in diesem Strahlengang einer Kamera zuzuführen.

Aus der US 5,867,309 A ist ein Stereomikroskop bekannt, bei dem ein an einem Display angezeigtes Bild mittels eines Blendenrads schnell alternierend in einen ersten und einen zweiten Beobachtungsstrahlengang eingespiegelt wird, um einen Stereoeindruck zu vermitteln.

Die US 2001/0010592 A1 beschreibt ein Operationsmikroskop mit einer linearbeweglichen Shutter-Anordnung, die es ermöglicht, einer Beobachtungsperson eine Display-Anzeige wahlweise in Überlagerung zu dem Bild des Objektbereichs, nur das Bild des Objektbereichs oder die Display-Anzeige zu visualisieren.

Hiervon ausgehend ist es eine Aufgabe der Erfindung, ein kompakt aufgebautes schaltbares Ein- und/oder Auskoppelmodul bereitzustellen, mit dem in einem optischen Instrument, z. B. in einem Operationsmikroskop, ein an einer ortsfesten optischen Schnittstelle zugeführter Strahlengang wahlweise in einen ersten oder in einen zweiten stereoskopischen Teilstrahlengang eingekoppelt und/oder mit dem ein wahlweise aus dem ersten oder dem zweiten stereoskopischen Teilstrahlengang ausgekoppelter Strahlengang an einer ortsfesten optischen Schnittstelle zur Verfügung gestellt werden kann.

Diese Aufgabe wird durch ein Einkoppelmodul mit den Merkmalen der Ansprüche 1, 5 sowie 10 und ein Auskoppelmodul mit den Merkmalen des Anspruchs 2, 6 sowie 11 gelöst. Vorteilhafte Ausführungsformen der Erfindung sind in den abhängigen Ansprüchen angegeben.

Operationsmikroskope werden oft von Gruppen von Operateuren eingesetzt, bei denen einige Operateure ein rechtes Führungsauge haben und andere ein linkes. Je nachdem, ob sich das Führungsauge links oder rechts befindet, ist für die Operateure das monokulare Einspiegeln von Daten entweder in den linken oder den rechten stereoskopischen Teilbeobachtungsstrahlengang wünschenswert.

Wenn gefordert wird, dass die in einem Operationsmikroskop eingespiegelten Bilddaten einen Bezug zur Beobachtung haben, z. B. wenn dem Operateur in Überlagerung zu dem in dem Operationsmikroskop sichtbaren Operationsbereich Bilder zur Anzeige gebracht werden, deren Lage, Größe und Orientierung an das von dem Betrachter wahrgenommene Bild des Operationsbereichs angepasst wird, etwa für das Anzeigen der Orientierung des Patientenauges in der Ophthalmochirurgie (Eye-Tracking), ist es in einem Operationsmikroskop für das Vermeiden von Parallaxefehlern vorteilhaft, dass in dem Operationsmikroskop sowohl das Auskoppeln eines Strahlengangs aus einem stereoskopischen Teilbeobachtungsstrahlengang zu einer Kamera und einer damit verbundenen Einrichtung für die Bildauswertung als auch das Einkoppeln eines Strahlengangs mit Orts- und Orientierungsinformationen in ein und demselben stereoskopischen Teilbeobachtungsstrahlengang erfolgt.

Vor diesem Hintergrund ist es insbesondere bei Ophthalmo-Operationsmikroskopen wünschenswert, dass der stereoskopische Teilstrahlengang ausgewählt werden kann, aus dem ein Strahlengang zu einer Kamera ausgekoppelt und in den das Bild einer Displayanzeige eingekoppelt wird.

Eine Idee der Erfindung ist es deshalb auch, ein Operationsmikroskop für das stereoskopische Beobachten eines Operationsbereichs mit einem ersten und mit einem zweiten stereoskopischen Teilstrahlengang bereitzustellen, das ein Einkoppelmodul für das wahlweise Einkoppeln eines Strahlengangs enthält, der durch eine in Bezug auf das Operationsmikroskop ortsfeste optische Schnittstelle von einem Display zugeführt wird. Weiter ist es eine Idee der Erfindung, in einem solchen Operationsmikroskop ein Auskoppelmodul für das wahlweise Auskoppeln eines Strahlengangs aus einem ersten oder zweiten stereoskopischen Teilstrahlengang zu einer in dem Operationsmikroskop ortsfesten Schnittstelle vorzusehen.

Ein erfindungsgemäßes Einkoppelmodul und ein erfindungsgemäßes Auskoppelmodul hat deshalb eine einstellbare Optikbaugruppe für das Umschalten des Strahlengangs aus einer ersten Position in eine zweite Position und umgekehrt. Diese einstellbare Optikbaugruppe enthält mindestens ein vorzugsweise linearbeweglich verlagerbares optisches Element, das in der ersten Position und/oder der zweiten Position in dem ein- bzw. auszukoppelnden Strahlengang angeordnet ist.

Der Erfindung liegt dabei der Gedanke zugrunde, dass wenn für das Einkoppeln eines an einer optischen Schnittstelle zugeführten Strahlengangs in die beiden stereoskopischen Teilstrahlengänge mit einem eine einstellbare Optikbaugruppe aufweisenden Einkoppelmodul bei einem Operationsmikroskop unterschiedliche optische Weglängen in Kauf genommen werden, für das Einkoppelmodul ein sehr kompakter Aufbau ermöglicht wird. Entsprechendes gilt für das wahlweise Auskoppeln eines Strahlengangs aus dem ersten oder zweiten stereoskopischen Teilstrahlengang in einem Operationsmikroskop, um den ausgekoppelten Strahlengang an einer optischen Schnittstelle bereitzustellen.

Darüber hinaus ist es eine Idee der Erfindung, die Anzahl der bewegbaren Baugruppen in einem solchen Ein- bzw. Auskoppelmodul zu minimieren, um auf diese Weise die Herstellungskosten gering zu halten.

Dieses mindestens eine optische Element kann z. B. ein Spiegelelement sein, das den einzukoppelnden Strahlengang in der ersten Position und / oder der zweiten Position umlenkt.

Bevorzugt enthält die einstellbare Optikbaugruppe wenigstens auch ein weiteres Spiegelelement, wobei das verlagerbare Spiegelelement in der ersten Position in dem einzukoppelnden Strahlengang angeordnet ist und den Strahlengang zu dem weiteren Spiegelelement lenkt, das den Strahlengang dem ersten Strahlteiler für das Einkoppeln in den ersten stereoskopischen Teilstrahlengang zuführt, und das in der zweiten Position den einzukoppelnden Strahlengang freigibt, damit dieser für das Einkoppeln in den zweiten stereoskopischen Teilstrahlengang zu dem zweiten Strahlteiler gelangt.

In einem erfindungsgemäßen Ein- bzw. Auskoppelmodul gibt es vorteilhafter Weise einen die Optikbaugruppe aufnehmenden Trägerrahmen, in dem das weitere Spiegelelement an einer Justiereinrichtung gehalten ist, mit der das Spiegelelement für das Einstellen der optischen Achse des dem ersten Strahlteiler zugeführten Strahlengangs justiert werden kann. Bevorzugt ist die Justiereinrichtung hierfür z. B. derart ausgebildet, dass sich das Spiegelelement um eine erste Bewegungsachse und um eine von der ersten Bewegungsachse verschiedene zweite Bewegungsachse verlagern lässt.

Das Einkoppelmodul hat erfindungsgemäß eine Schnittstelle mit einer optischen Achse. Unter der optischen Achse einer Schnittstelle ist vorliegend die Symmetrieachse des Strahlengangs zu verstehen, der die Schnittstelle durchsetzt. In dem Einkoppelmodul gibt es einen den ersten Strahlteiler durchsetzenden eine optische Achse aufweisenden ersten Beobachtungskanal und einen den zweiten Strahlteiler durchsetzenden zweiten Beobachtungskanal, der ebenfalls eine optische Achse hat. Unter der optischen Achse eines Beobachtungskanals wird vorliegend die Symmetrieachse des in dem Beobachtungskanal geführten optischen Strahlengangs verstanden. Die optische Achse des ersten Beobachtungskanals und die optische Achse des zweiten Beobachtungskanals in dem Einkoppelmodul liegen dabei in einer gemeinsamen zu der optischen Achse der Schnittstelle senkrechten Ebene. Die optische Achse der Schnittstelle durchsetzt dann den ersten oder den zweiten Strahlteiler. Dabei kann die optische Achse der Schnittstelle die optische Achse des ersten oder des zweiten Beobachtungskanals insbesondere schneiden.

Das Einkoppelmodul kann aber auch einen den ersten Strahlteiler durchsetzenden, eine optische Achse aufweisenden ersten Beobachtungskanal und einen den zweiten Strahlteiler durchsetzenden eine optische Achse aufweisenden zweiten Beobachtungskanal haben, wobei die optische Achse des ersten Beobachtungskanals und die optische Achse des zweiten Beobachtungskanals in einer gemeinsamen zu der optischen Achse der Schnittstelle parallelen Ebene liegen.

Eine Idee der Erfindung ist es insbesondere, in dem Einkoppelmodul einen die Optikbaugruppe aufnehmenden Trägerrahmen vorzusehen, in dem das linearbeweglich verlagerbare Spiegelelement an einer Justiereinrichtung gehalten ist, mit der das Spiegelelement für das Einstellen der optischen Achse des dem ersten Strahlteiler und dem zweiten Strahlteiler zugeführten Strahlengangs justiert werden kann.

Darüber hinaus ist es eine Idee der Erfindung, dass die einstellbare Optikbaugruppe ein linearbeweglich verlagerbares Shutterelement für das wahlweise Abdecken des ersten Strahlteilers oder des zweiten Strahlteilers enthält. Insbesondere kann das Shutterelement als eine von einem an der optischen Schnittstelle zugeführten Strahlengang durchsetzte Lochblende ausgebildet sein.

Die einstellbare Optikbaugruppe kann auch ein mit dem Spiegelelement bewegungsgekoppeltes weiteres linearbeweglich verlagerbares Spiegelelement enthalten, wobei das Spiegelelement und das weitere Spiegelelement aus einer ersten Position in eine zweite Position und umgekehrt bewegt werden können. Das Spiegelelement und das weitere Spiegelelement sind dabei in der ersten Position in dem einzukoppelnden Strahlengang angeordnet und lenken den Strahlengang zu dem ersten Strahlteiler. Sie geben den einzukoppelnden Strahlengang in der zweiten Position frei, damit dieser für das Einkoppeln in den zweiten stereoskopischen Teilstrahlengang zu dem zweiten Strahlteiler gelangt.

Das Einkoppelmodul kann dabei ebenfalls einen die Optikbaugruppe aufnehmenden Trägerrahmen aufweisen, in dem das Spiegelelement und/oder das weitere Spiegelelement an einer Justiereinrichtung gehalten ist, mit der das Spiegelelement und/oder das weitere Spiegelelement für das Einstellen der optischen Achse des dem ersten Strahlteiler zugeführten Strahlengangs justiert werden kann. Die Justiereinrichtung ermöglicht hierfür z. B., dass das Spiegelelement um eine erste Bewegungsachse und um eine von der ersten Bewegungsachse verschiedene zweite Bewegungsachse verlagert werden kann.

Von Vorteil ist es, wenn die einstellbare Optikbaugruppe ein mit dem ersten Spiegelelement und dem weiteren Spiegelelement bewegungsgekoppeltes linearbeweglich verlagerbares Shutterelement enthält, das in der ersten Position des Spiegelelements und des weiteren Spiegelelements den zweiten Strahlteiler abdeckt und das in der zweiten Position des ersten Spiegelelements und des weiteren Spiegelelements den ersten Strahlteiler abdeckt.

Es ist möglich, das linearbeweglich verlagerbare Shutterelement auch als eine Lochblende mit einer Blendenöffnung auszubilden, die mit einem an der optischen Schnittstelle zugeführten Strahlengang durchsetzt ist.

Das optische Element kann als eine in der ersten Position und in der zweiten Position von dem Strahlengang durchsetztes linearbeweglich verlagerbares Shutterelement, insbesondere in Form einer Lochblende mit einer Blendenöffnung ausgebildet sein, wobei die einstellbare Optikbaugruppe einen dritten Strahlteiler enthält, der den an der optischen Schnittstelle zuführbaren einzukoppelnden Strahlengang in einen ersten Teilstrahlengang und in einen zweiten Teilstrahlengang aufteilt, wobei der erste Teilstrahlengang zu dem zweiten Strahlteiler und der zweite Teilstrahlengang zu einem Spiegelelement geführt ist, das den zweiten Teilstrahlengang zu dem ersten Strahlteiler umlenkt, wobei die Lochblende in der ersten Position den ersten Strahlteiler abdeckt und die Blendenöffnung den zweiten Strahlteiler für den ersten Teilstrahlengang freigibt und die Lochblende in der zweiten Position den zweiten Strahlteiler abdeckt und die Blendenöffnung den ersten Strahlteiler für den zweiten Teilstrahlengang freigibt.

Diese Optikbaugruppe hat erfindungsgemäß eine Schnittstelle mit einer optischen Achse und enthält einen den ersten Strahlteiler durchsetzenden eine optische Achse aufweisenden ersten Beobachtungskanal und einen den zweiten Strahlteiler durchsetzenden eine optische Achse aufweisenden zweiten Beobachtungskanal. Die optische Achse des ersten Beobachtungskanals und die optische Achse des zweiten Beobachtungskanals liegen dabei in einer gemeinsamen zu der optischen Achse der Schnittstelle parallelen oder senkrechten Ebene. Wenn die optische Achse der Schnittstelle zu der von den optischen Achsen des ersten und des zweiten Beobachtungskanals aufgespannten Ebene senkrecht ist, so durchsetzt die optische Achse der Schnittstelle bevorzugt die optische Achse des ersten oder des zweiten Strahlteilers. Dabei schneidet dann die optische Achse der Schnittstelle die optische Achse des ersten oder des zweiten Beobachtungskanals.

Von Vorteil ist es, wenn das Einkoppelmodul einen die Optikbaugruppe aufnehmenden Trägerrahmen hat, in dem das Spiegelelement an einer Justiereinrichtung gehalten ist, mit der das Spiegelelement für das Einstellen der optischen Achse des dem ersten Strahlteiler zugeführten Strahlengangs justiert werden kann.

Auch ein erfindungsgemäßes Auskoppelmodul hat bevorzugt neben dem von einer ersten Position in eine zweite Position linearbeweglich verlagerbaren Spiegelelement, das den ausgekoppelten Strahlengang in der ersten und/oder der zweiten Position umlenkt, in der einstellbaren Optikbaugruppe ein weiteres Spiegelelement. Das weitere Spiegelelement führt dabei einen mit dem ersten Strahlteiler ausgekoppelten stereoskopischen Teilstrahlengang dem weiteren Spiegelelement zu, wobei das linearbeweglich verlagerbare Spiegelelement in der ersten Position den mit dem ersten Strahlteiler ausgekoppelten Teilstrahlengang von dem weiteren Spiegelelement erhält und den Strahlengang der optischen Schnittstelle zuführt und in der zweiten Position den mit dem zweiten Strahlteiler ausgekoppelten Teilstrahlengang für das Hindurchtreten zu der optischen Schnittstelle freigibt.

Auch das Auskoppelmodul kann einen die Optikbaugruppe aufnehmenden Trägerrahmen haben, in dem das weitere Spiegelelement an einer Justiereinrichtung gehalten ist, mit der das Spiegelelement für das Einstellen der optischen Achse des von dem ersten Strahlteiler zugeführten Strahlengangs justiert werden kann.

Das Auskoppelmodul weist eine Schnittstelle mit einer optischen Achse auf, an die eine Kamera angeschlossen werden kann. Durch das Auskoppelmodul ist ein den ersten Strahlteiler durchsetzender eine optische Achse aufweisender erster Beobachtungskanal und ein den zweiten Strahlteiler durchsetzender eine optische Achse aufweisender zweiter Beobachtungskanal geführt. Die optische Achse des ersten Beobachtungskanals und die optische Achse des zweiten Beobachtungskanals liegen dabei in einer gemeinsamen zu der optischen Achse der Schnittstelle senkrechten Ebene.

Unter der optischen Achse eines Beobachtungskanals wird auch hier vorliegend die Symmetrieachse des in dem Beobachtungskanal geführten optischen Strahlengangs verstanden. Unter der optischen Achse einer Schnittstelle ist vorliegend ebenfalls die Symmetrieachse des Strahlengangs zu verstehen, der die Schnittstelle durchsetzt.

Alternativ hierzu ist es auch möglich, dass die optische Achse des ersten Beobachtungskanals und die optische Achse des zweiten Beobachtungskanals in einer gemeinsamen zu der optischen Achse der Schnittstelle parallelen Ebene liegen.

Auch das Auskoppelmodul weist erfindungsgemäß einen die Optikbaugruppe aufnehmenden Trägerrahmen auf, in dem das Spiegelelement und/oder das weitere Spiegelelement an einer Justiereinrichtung gehalten ist, mit der das Spiegelelement und/oder das weitere Spiegelelement für das Einstellen der optischen Achse des dem ersten Strahlteiler zugeführten Strahlengangs justiert werden kann, etwa indem das Spiegelelement um eine erste Bewegungsachse und um eine von der ersten Bewegungsachse verschiedene zweite Bewegungsachse verlagert wird.

Von Vorteil ist es, wenn dieses Auskoppelmodul einen die Optikbaugruppe aufnehmenden Trägerrahmen hat, in dem auch das erste linearbeweglich verlagerbare Spiegelelement an einer Justiereinrichtung gehalten ist, mit der das Spiegelelement für das Einstellen der optischen Achse des dem ersten Strahlteiler und dem zweiten Strahlteiler zugeführten Strahlengangs justiert werden kann.

Die einstellbare Optikbaugruppe kann insbesondere ein mit dem linearbeweglich verlagerbaren Spiegelelement bewegungsgekoppeltes linearbeweglich verlagerbares Shutterelement für das wahlweise Abdecken des ersten oder des zweiten Strahlteilers enthalten. Dabei ist es insbesondere möglich, dieses Shutterelement als eine von einem an der optischen Schnittstelle zugeführten Strahlengang durchsetzte Lochblende auszubilden.

Die einstellbare Optikbaugruppe kann auch ein mit dem Spiegelelement bewegungsgekoppeltes weiteres linearbeweglich verlagerbares Spiegelelement enthalten, wobei das Spiegelelement und das weitere Spiegelelement aus einer ersten Position in eine zweite Position und umgekehrt bewegt werden können, wobei das Spiegelelement und das weitere Spiegelelement in der ersten Position in dem auszukoppelnden Strahlengang angeordnet sind und den Strahlengang von dem ersten Strahlteiler zu der optischen Schnittstelle lenken, und wobei das Spiegelelement und das weitere Spiegelelement in der zweiten Position den auszukoppelnden Strahlengang freigeben, damit dieser von dem zweiten Strahlteiler zu der optischen Schnittstelle gelangt.

Erfindungsgemäß enthält die einstellbare Optikbaugruppe hier ein mit dem ersten Spiegelelement und dem weiteren Spiegelelement bewegungsgekoppeltes linearbeweglich verlagerbares Shutterelement, das in der ersten Position des Spiegelelements und des weiteren Spiegelelements den zweiten Strahlteiler abdeckt und in der zweiten Position des ersten Spiegelelements und des weiteren Spiegelelements den ersten Strahlteiler abdeckt.

Das verlagerbare optische Element ist als ein Shutterelement, insbesondere als ein Shutterelement in Form einer Lochblende mit einer lochförmigen Blendenöffnung ausgebildet, wobei die einstellbare Optikbaugruppe ein Spiegelelement enthält, das einen mittels des ersten Strahlteilers aus dem ersten stereoskopischen Teilstrahlengang ausgekoppelten Strahlengang zu einem weiteren Strahlteiler lenkt, der den Strahlengang der optischen Schnittstelle zuführt, wobei der weitere Strahlteiler einen mittels des zweiten Strahlteilers aus dem zweiten stereoskopischen Teilstrahlengang ausgekoppelten Strahlengang der optischen Schnittstelle zuführt, und wobei das Shutterelement in der ersten Position den mittels des ersten Strahlteilers ausgekoppelten Strahlengang sperrt und den mittels des zweiten Strahlteilers ausgekoppelten Strahlengang freigibt und in der zweiten Position den mittels des ersten Strahlteilers ausgekoppelten Strahlengang freigibt und den mittels des zweiten Strahlteilers ausgekoppelten Strahlengang sperrt.

Das Auskoppelmodul kann einen den ersten Strahlteiler durchsetzenden, eine optische Achse aufweisenden ersten Beobachtungskanal und einen den zweiten Strahlteiler durchsetzenden, eine optische Achse aufweisenden zweiten Beobachtungskanal haben, wobei die optischen Achsen des ersten und zweiten Beobachtungskanals in einer gemeinsamen Ebene liegen, und wobei die optische Schnittstelle für das Bereitstellen des Strahlengangs eine zu der gemeinsamen Ebene parallele optische Achse oder eine zu der gemeinsamen Ebene senkrechte optische Achse hat. Wenn die optische Achse der Schnittstelle zu der von den optischen Achsen des ersten und des zweiten Beobachtungskanals aufgespannten Ebene senkrecht ist, so durchsetzt die optische Achse der Schnittstelle bevorzugt die optische Achse des ersten oder des zweiten Strahlteilers. Dabei schneidet dann die optische Achse der Schnittstelle die optische Achse des ersten oder des zweiten Beobachtungskanals.

Von Vorteil ist es auch hier, wenn das Auskoppelmodul einen die Optikbaugruppe aufnehmenden Trägerrahmen enthält, in dem das Spiegelelement an einer Justiereinrichtung gehalten ist, mit der das Spiegelelement für das Einstellen der optischen Achse des von dem ersten Strahlteiler zugeführten Strahlengangs justiert werden kann, bevorzugt durch Verlagern um eine erste Bewegungsachse und um eine von der ersten Bewegungsachse verschiedene zweite Bewegungsachse.

Die Erfindung erstreckt sich auch auf ein Modul für das wahlweise Einkoppeln eines Strahlengangs in einen ersten oder zweiten stereoskopischen Teilstrahlengang in einem Operationsmikroskop und das wahlweise Auskoppeln eines Strahlengangs aus einem ersten oder zweiten stereoskopischen Teilstrahlengang in einem Operationsmikroskop. Dieses Modul kann einen verstellbaren Shutter für das wahlweise Freigeben und Unterbrechen des ersten stereoskopischen Teilstrahlengangs und/oder des zweiten stereoskopischen Teilstrahlengangs zwischen dem Einkoppelmodul und dem Auskoppelmodul aufweisen.

Ein erfindungsgemäßes Ein- bzw. Auskoppelmodul kann im Wesentlichen aus Planoptiken aufgebaut sein. Es lässt sich deshalb in kompakten Bauformen mit geringem Justageaufwand kostengünstig herstellen. Aufgrund der kompakten Bauform eignet sich ein solches Ein- bzw. Auskoppelmodul insbesondere für den Einsatz in unterschiedlichen Operationsmikroskopen. Eine Idee der Erfindung besteht deshalb auch darin, ein Ein- bzw. Auskoppelmodul als eine modulare Baugruppe bzw. Plattform-Baugruppe zu gestalten, die für den Einsatz in unterschiedlichen Operationsmikroskoptypen geeignet ist.

Die Erfindung ermöglicht damit z. B., dass in einem Operationsmikroskop, das nur ein Display für die Dateneinspiegelung und lediglich eine Kamera für das Erfassen von Bildern des Objektbereichs enthält, wahlweise ein aus dem ersten oder zweiten stereoskopischen Teilstrahlengang ausgekoppelter Strahlengang der Kamera zugeführt und die mittels des Displays angezeigte Bildinformation in den ersten oder zweiten stereoskopischen Teilstrahlengang eingekoppelt werden kann.

Mit einem erfindungsgemäßen Ein- bzw. Auskoppelmodul ist auch ein einfaches elektromotorisches Umschalten zwischen einem ersten und einem zweiten stereoskopischen Teilstrahlengang möglich. Damit kann z. B. bei einem Operationsmikroskop im laufenden Operationsbetrieb das Einkoppeln der Anzeigeinformation eines einzigen Displays wahlweise in dem ersten oder dem zweiten stereoskopischen Teilstrahlengang erfolgen und es ist möglich, das Bild des Objektbereichs entweder aus dem ersten oder dem zweiten stereoskopischen Teilstrahlengang auszukoppeln, um ihn einer Kamera zuzuführen.

Die Erfindung erstreckt sich auch auf ein Operationsmikroskop für das stereoskopische Beobachten eines Operationsbereichs mit einem ersten und mit einem zweiten stereoskopischen Teilstrahlengang, das ein umschaltbares System zur Strahlvertauschung und Bildumkehr für das wahlweise Vertauschen des ersten und zweiten stereoskopischen Teilstrahlengangs und das Einstellen einer Bildumkehr für das einer Beobachtungsperson an einem linken und rechten Okulareinblick bereitgestellte Bild eines Objektbereichs enthält. Das Operationsmikroskop enthält ebenfalls ein Ein- und Auskoppelmodul für das wahlweise Einkoppeln eines Strahlengangs von einer Displayanzeige in den ersten oder zweiten stereoskopischen Teilstrahlengang und das wahlweise Auskoppeln eines Strahlengangs aus dem ersten oder zweiten stereoskopischen Teilstrahlengang zu einer Kamera. Das System zur Strahlvertauschung und Bildumkehr ist dabei mit dem Ein- und Auskoppelmodul über eine Kopplungseinrichtung verbunden, die bei einem Umschalten des Systems zur Strahlvertauschung und Bildumkehr das Ein- und/oder das Auskoppelmodul umschaltet, um den Strahlengang von der Displayanzeige in einen anderen der beiden stereoskopischen Teilstrahlengänge einzukoppeln bzw. um der Kamera einen anderen der beiden stereoskopischen Teilstrahlengänge zuzuführen. Auf diese Weise lässt sich gewährleisten, dass für eine Beobachtungsperson bei dem Operationsmikroskop Bildinformation aus demjenigen Beobachtungsstrahlengang ausgekoppelt und in denjenigen Beobachtungsstrahlenggang eingekoppelt wird, der einem bestimmten Auge der Beobachtungsperson vor dem Umschalten des Systems zur Strahlvertauschung und Bildumkehr zugeführt wird. Damit kann insbesondere sichergestellt werden, dass das Führungsauge der Beobachtungsperson immer die in einen bestimmten stereoskopischen Teilstrahlengang vor dem System zur Strahlvertauschung und Bildumkehr eingespiegelte Displayinformation unabhängig von dem Schaltzustand dieses Systems erhält.

Im Folgenden wird die Erfindung anhand der in der Zeichnung in schematischer Weise dargestellten Ausführungsbeispiele näher erläutert.

Es zeigen:
- Fig. 1: ein Operationsmikroskop für das stereoskopische Beobachten eines Objektbereichs mit einem Vergrößerungssystem und einer als Ein- und Auskoppelmodul ausgebildeten Modulbaugruppe;
- Fig. 2: und Fig. 3 eine perspektivische Ansicht der Modulbaugruppe mit dem Vergrößerungssystem;
- Fig. 4: die Modulbaugruppe in dem Operationsmikroskop in einer ersten Einstellung;
- Fig. 5: die Modulbaugruppe in dem Operationsmikroskop in einer zweiten Einstellung;
- Fig. 6: und Fig. 7 das Auskoppelmodul der Modulbaugruppe;
- Fig. 8: und Fig. 9 das Einkoppelmodul der Modulbaugruppe;
- Fig. 10: eine Justiereinrichtung für ein Spiegelelement in der Modulbaugruppe;
- Fig. 11: eine weitere Modulbaugruppe für den Einsatz in einem Operationsmikroskop mit einem Shutter;
- Fig. 12: bis Fig. 15 eine Teilansicht der weiteren Modulbaugruppe mit unterschiedlichen Shutter-Einstellungen;
- Fig. 16: und Fig. 17 eine weitere Modulbaugruppe für den Einsatz in einem Operationsmikroskop mit einem Shutter;
- Fig. 18: bis Fig. 21 eine weitere Modulbaugruppe für den Einsatz in einem Operationsmikroskop mit einem Shutter;
- Fig. 22: bis Fig. 27 verschiedene weitere Einkoppelmodule für ein Operationsmikroskop in unterschiedlichen Einstellungen;
- Fig. 28: bis Fig. 31 verschiedene weitere Auskoppelmodule für ein Operationsmikroskop in unterschiedlichen Einstellungen;
- Fig. 32: ein Operationsmikroskop für das stereoskopische Beobachten eines Objektbereichs mit einer als Ein- und Auskoppelmodul ausgebildeten Modulbaugruppe und mit einem System zur Strahlvertauschung und Bildumkehr in einer ersten Einstellung; und
- Fig. 33: das in der Fig. 32 gezeigte Operationsmikroskop mit dem System zur Strahlvertauschung und Bildumkehr in einer zweiten Einstellung.

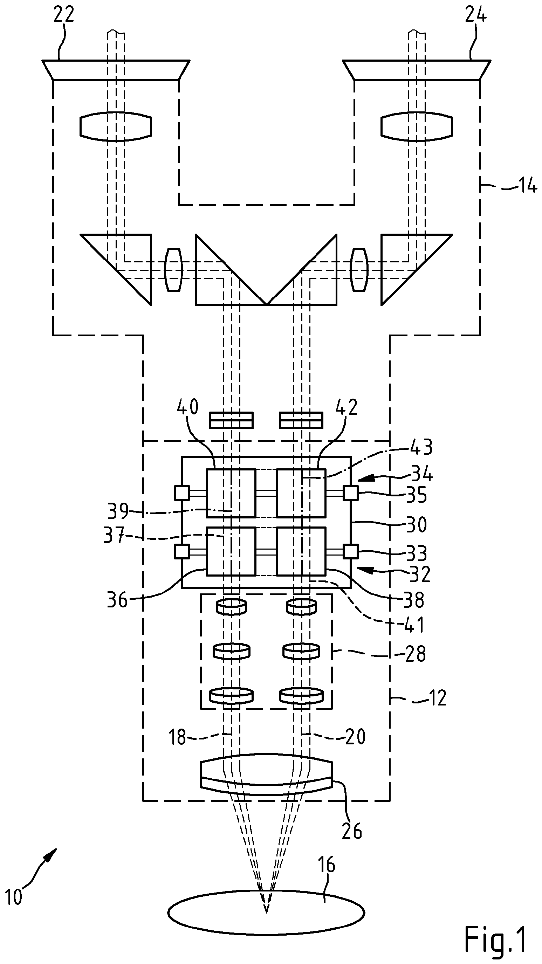

Das in der Fig. 1 gezeigte Operationsmikroskop 10 ist an einer nicht weiter dargestellten Trägervorrichtung aufgenommen und hat einen Grundkörper 12 und einen Binokulartubus 14. Das Operationsmikroskop 10 ist ein optisches Instrument und ermöglicht einer Beobachtungsperson mit einem ersten stereoskopischen Teilstrahlengang 18 und einem zweiten stereoskopischen Teilstrahlengang 20 das vergrößerte Betrachten eines Objektbereichs 16 an einem linken und rechten Okulareinblick 22, 24.

Die stereoskopischen Teilstrahlengänge 18, 20 des Operationsmikroskops 10 durchsetzen ein in dem Grundkörper 12 aufgenommenes Mikroskop-Hauptobjektiv 26 und sind mit einem parallelen Abbildungsstrahlengang durch ein in dem Grundkörper 12 aufgenommenes Vergrößerungssystem 28 geführt. Das Vergrößerungssystem 28 hat ein Zoomsystem. Auf der dem Mikroskop-Hauptobjektiv 26 abgewandten Seite des Vergrößerungssystems 28 ist in dem Grundkörper 12 eine Modulbaugruppe 30 angeordnet. Die Modulbaugruppe 30 befindet sich in dem parallelen Abbildungsstrahlengang des Operationsmikroskops 10. Die Modulbaugruppe 30 ist ein Ein- und Auskoppelmodul. Die Modulbaugruppe 30 enthält ein Einkoppelmodul 34 für das wahlweise Einkoppeln eines an einer ersten optischen Schnittstelle (nicht gezeigt) zugeführten Strahlengangs in den ersten oder den zweiten stereoskopischen Teilstrahlengang 18, 20. Weiter enthält die Modulbaugruppe 30 ein Auskoppelmodul 32 für das wahlweise Auskoppeln eines Strahlengangs aus dem ersten oder dem zweiten stereoskopischen Teilstrahlengang 18, 20 zu einer von der ersten optischen Schnittstelle verschiedenen zweiten optischen Schnittstelle (nicht gezeigt).

Das Einkoppelmodul 34 weist eine in einem Trägerrahmen 33 angeordnete einstellbare Optikbaugruppe auf und enthält einen in dem ersten stereoskopischen Teilstrahlengang 18 positionierten ersten Strahlteiler 36 sowie einen in dem zweiten stereoskopischen Teilstrahlengang 20 positionierten zweiten Strahlteiler 38. Der erste Strahlteiler 36 ist in dem Operationsmikroskop 10 von einem Beobachtungskanal 37 durchsetzt, der eine optische Achse 39 hat und in dem der erste stereoskopische Teilstrahlengang 18 geführt ist. Durch den zweiten Strahlteiler 38 in dem Operationsmikroskop 10 verläuft ein Beobachtungskanal 41 mit einer optischen Achse 43, in dem der zweite stereoskopische Teilstrahlengang 20 geführt ist.

Auch das Auskoppelmodul 32 hat eine in einem Trägerrahmen 35 angeordnete einstellbare Optikbaugruppe und weist einen in dem ersten stereoskopischen Teilstrahlengang 18 angeordneten ersten Strahlteiler 40 auf, durch den der Beobachtungskanal 37 mit der optischen Achse 39 geführt ist, und enthält einen zweiten Strahlteiler 42, der sich in dem zweiten stereoskopischen Teilstrahlengang 20 befindet und durch den der Beobachtungskanal 41 mit der optischen Achse 43 verläuft.

Zu bemerken ist, dass die Strahlteiler 36, 38 in dem Einkoppelmodul 34 einerseits und die Strahlteiler 40, 42 in dem Auskoppelmodul 32 andererseits bevorzugt als ein jeweils einteiliges, zusammenhängendes Bauelement ausgebildet sind. Diese Maßnahme verringert den Aufwand für eine binokulare Justage und reduziert auch die Anforderungen an Bauteiltoleranzen. In einer modifizierten Ausführungsform können die Strahlteiler 36, 38 bzw. 40, 42 allerdings grundsätzlich auch als räumlich voneinander getrennte Teilerwürfel ausgebildet sein.

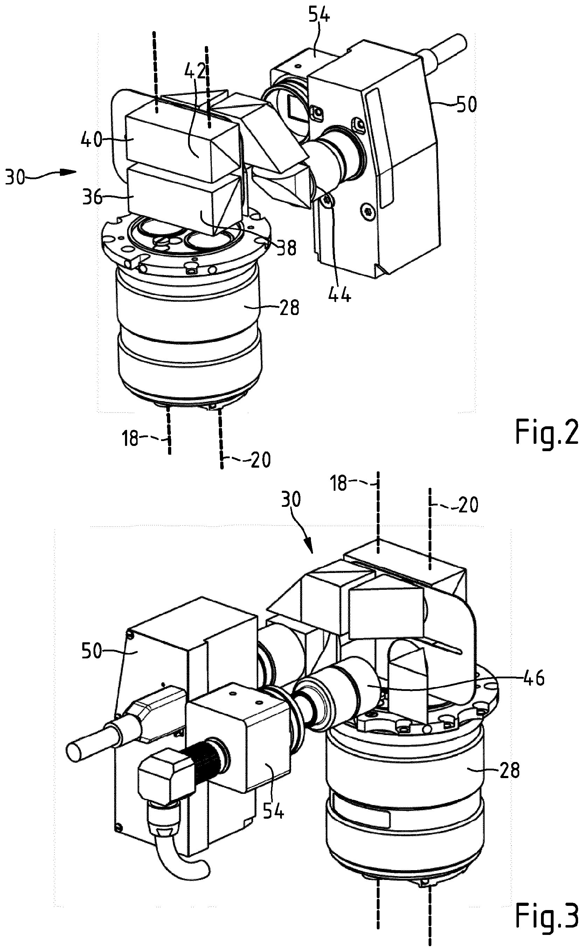

Die Fig. 2 und Fig. 3 zeigen die Modulbaugruppe 30 mit der ersten optischen Schnittstelle 44 und der zweiten optischen Schnittstelle 46 und mit dem Vergrößerungssystem 28 in zwei unterschiedlichen perspektivischen Ansichten. Die Strahlteiler 36, 38 einerseits und die Strahlteiler 40, 42 andererseits sind hier einstückig als ein zusammenhängender Prismenquader gestaltet. An der optischen Schnittstelle 44 des Einkoppelmoduls 34 wird dem ersten bzw. zweiten stereoskopischen Teilstrahlengang 18, 20 in dem Operationsmikroskop 10 die Anzeige eines in einem Displaymodul 50 angeordneten Displays zugeführt. An der optischen Schnittstelle 46 des Auskoppelmoduls 32 ist ein aus dem ersten bzw. zweiten stereoskopischen Teilstrahlengang 18, 20 in dem Operationsmikroskop 10 ausgekoppelter Strahlengang zu einer Kamera 54 bereitgestellt.

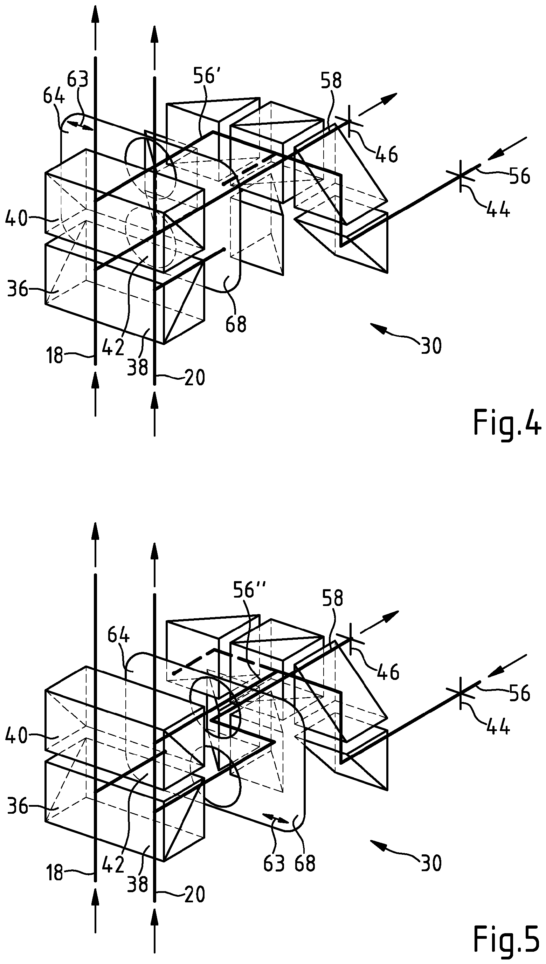

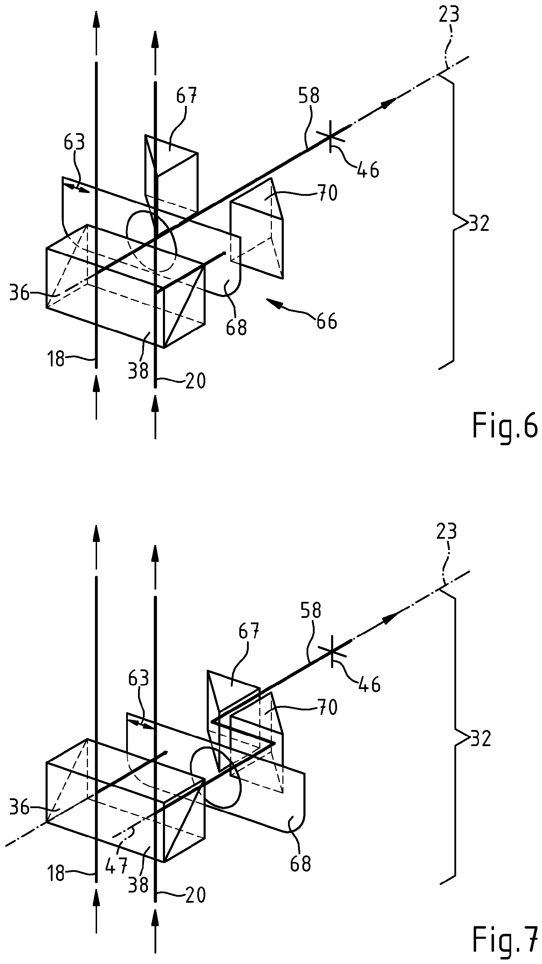

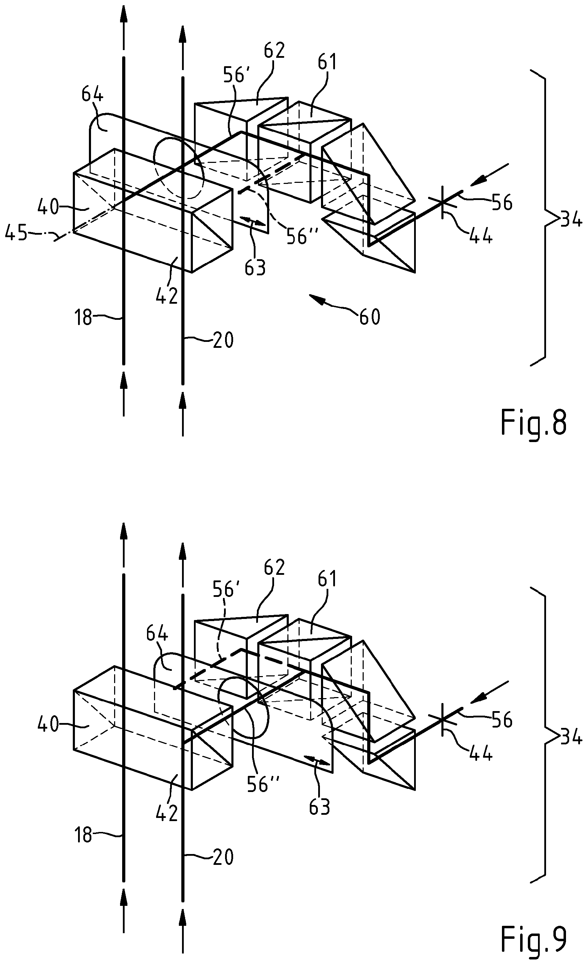

Die Fig. 4 zeigt die Modulbaugruppe 30 mit den stereoskopischen Teilstrahlengängen 18, 20 und einem an der optischen Schnittstelle 44 zugeführten Strahlengang 56, der mit dem Strahlengang 56' in den ersten stereoskopischen Teilstrahlengang 18 eingekoppelt wird, sowie einem aus dem ersten stereoskopischen Teilstrahlengang 20 ausgekoppelten Strahlengang 58, der an der optischen Schnittstelle 46 bereitgestellt wird. In der Fig. 5 ist die Modulbaugruppe 30 in einer zweiten Einstellung mit einem eingekoppelten Strahlengang 56" und einem ausgekoppelten Strahlengang 58 gezeigt. Die Fig. 6 und Fig. 7 zeigen das Auskoppelmodul 32 in der Modulbaugruppe 30 in der ersten und zweiten Einstellung. In der Fig. 8 und Fig. 9 ist das Einkoppelmodul 34 der Modulbaugruppe 30 in einer ersten und einer zweiten Einstellung abgebildet.

Das Auskoppelmodul 32 hat eine einstellbare Optikbaugruppe 66 mit einem als Spiegelelement 67 in Form eines Umlenkprismas ausgebildeten linearbeweglich verlagerbaren optischen Element, mit einem weiteren optischen Element in Form einer als ein Shutterelement wirkenden linearbeweglich angeordneten Lochblende 68, und mit einem weiteren Umlenkprisma, das wiederum als ein Spiegelelement 70 wirkt. Die optische Schnittstelle 46 des Auskoppelmoduls 32 hat eine optische Achse 23, die den Strahlteiler 36 durchsetzt und den ersten stereoskopischen Beobachtungsstrahlengang 18 schneidet.

Das Einkoppelmodul 34 weist eine einstellbare Optikbaugruppe 60 auf, die einen Strahlteiler 61 in Form eines Teilerwürfels umfasst und die ein als Umlenkprisma ausgebildetes Spiegelelement 62 sowie eine linearbeweglich angeordnete Lochblende 64 als ein weiteres optisches Element enthält.

Das Spiegelelement 67 ist über einen Kopplungsmechanismus (nicht gezeigt) mit der Lochblende 68 bewegungsgekoppelt. Außerdem ist hier die linearbewegliche Lochblende 68 mit der Lochblende 64 bewegungsgekoppelt. Hierfür sind in der Modulbaugruppe 30 die Lochblende 68 und die Lochblende 64 als ein zusammenhängendes Blendenbauteil ausgebildet.

Ein an der optischen Schnittstelle 44 zugeführter Strahlengang 56 wird mittels des Strahlteilers 61 in einen ersten Teilstrahlengang 56' und einen zweiten Teilstrahlengang 56" aufgeteilt. Das Spiegelelement 62 lenkt den Teilstrahlengang 56' zu dem in dem zweiten stereoskopischen Teilstrahlengang 18 angeordneten Strahlteiler 40. Der Teilstrahlengang 56' ist zu dem in dem ersten stereoskopischen Teilstrahlengang 20 angeordneten ersten Strahlteiler 42 geführt. Die Lochblende 64 wirkt als ein Shutterelement und kann in der Richtung des Doppelpfeils 63 verlagert werden. Damit ist es möglich, wahlweise, wie in der Fig. 4 und der Fig. 8 gezeigt, den der optischen Schnittstelle 44 zugeführten Strahlengang 56 entweder mit dem Teilstrahlengang 56' in den ersten stereoskopischen Teilstrahlengang 18 einzukoppeln, wobei der Teilstrahlengang 56" dann mittels der Lochblende 64 unterbrochen ist, oder, wie in der Fig. 5 und der Fig. 9 zu sehen, den der optischen Schnittstelle zugeführten Strahlengang mit dem Teilstrahlengang 56" in den zweiten stereoskopischen Teilstrahlengang 20 einzukoppeln, wobei der erste Teilstrahlengang 56' dann mittels der Lochblende 64 unterbrochen ist.

Indem in der einstellbare Optikbaugruppe 66 des Auskoppelmoduls 32 das Umlenkprisma 67 und die Lochblende 68 in der Richtung des Doppelpfeils 63 verlagert werden, ist es möglich, an der Schnittstelle 46 wahlweise einen Strahlengang 58 bereitzustellen, der mittels des Strahlteilers 36 oder mittels des Strahlteilers 38 aus dem ersten stereoskopischen Teilstrahlengang 18 bzw. dem zweiten stereoskopischen Teilstrahlengang 20 ausgekoppelt wird.

Dabei wird in der ersten Einstellung der Lochblende 68 und des Umlenkprismas 67 der mittels des Strahlteilers 36 aus dem ersten stereoskopischen Teilstrahlengang 18 ausgekoppelte Strahlengang 58 direkt zu der optischen Schnittstelle 46 geführt. In der zweiten Einstellung der Lochblende 68 und des Umlenkprismas 67 wird der mittels des Strahlteilers 38 aus dem zweiten stereoskopischen Teilstrahlengang 20 ausgekoppelte Strahlengang von dem Umlenkprisma 70 auf das Umlenkprisma 67 gelenkt, das den ausgekoppelten Strahlengang dann zu der optischen Schnittstelle 46 spiegelt.

Das Umlenkprisma 67 in dem Einkoppelmodul 34 und das Umlenkprisma 70 in dem Auskoppelmodul 32 sind in den Trägerrahmen 33, 35 der Optikbaugruppen 60, 66 jeweils an einer Justiereinrichtung gehalten, mit der die optische Achse 45, 47 des mittels des Spiegelelements 62, 70 umgelenkten Strahlengangs um zwei zueinander orthogonale Bewegungsachsen justiert werden kann.

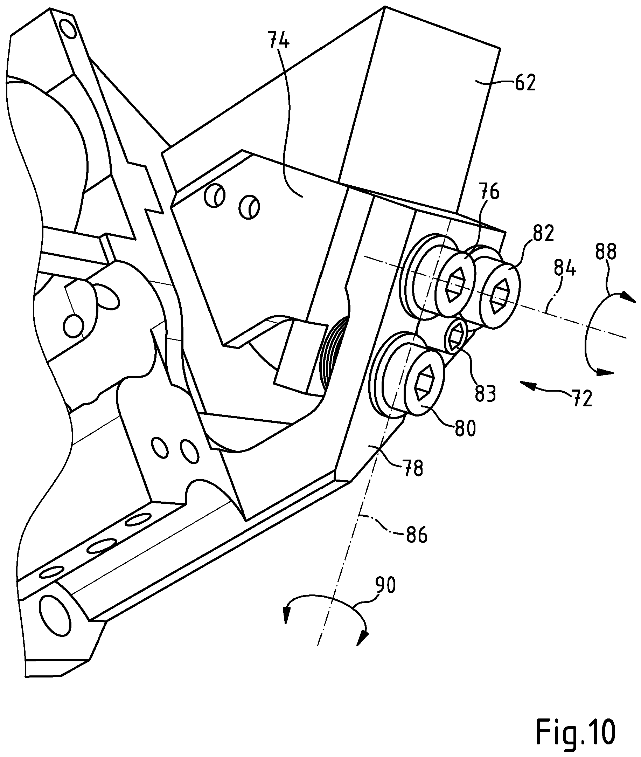

Die Fig. 10 zeigt die Justiereinrichtung 72 für das Spiegelelement 62. Das Spiegelelement 62 ist in einem Halter 74 festgelegt, der mit einer als Gelenkschraube wirkenden Befestigungsschraube 76 an einem an dem Trägerrahmen 35 der in der Fig. 1 gezeigten Optikbaugruppe angeschlossenen Träger 78 befestigt ist. An dem Träger 78 sind Justierschrauben 80, 82 aufgenommen. Durch Verstellen der Justierschrauben 80, 82 kann der Halter 74 mit dem daran angeordneten Spiegelelement 62 relativ zu dem Träger 78 um eine erste Bewegungsachse 84 und um eine zu der ersten Bewegungsachse 84 senkrechte weitere Bewegungsachse 86 in den mittels der Doppelpfeile 88, 90 angedeuteten Richtungen geschwenkt bzw. gekippt werden. Für das Fixieren einer mit den Justierschrauben 80, 82 vorgenommenen Einstellung gibt es in der Justiereinrichtung 72 eine Madenschraube 83.

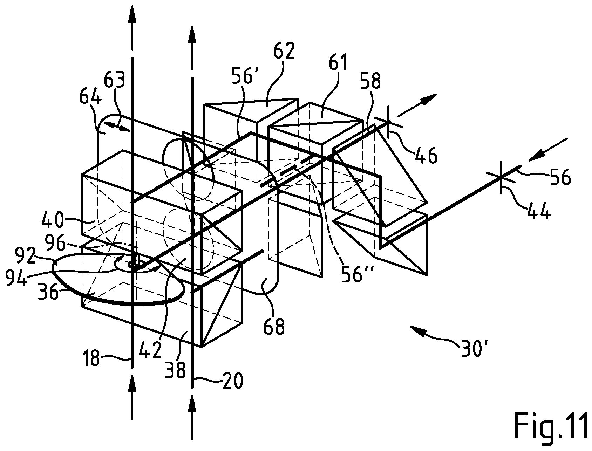

Die Fig. 11 zeigt eine der vorstehend beschriebenen Modulbaugruppe 30 entsprechende Modulbaugruppe 30' für den Einsatz in einem stereoskopischen Operationsmikroskop. Soweit die optischen Elemente der Modulbaugruppe 30' und die optischen Elemente der Modulbaugruppe 30 identisch sind, haben diese in der Fig. 11 die gleichen Bezugszeichen wie in den Fig. 4 bis Fig. 9.

Die Modulbaugruppe 30' enthält einen drehbeweglich gelagerten Shutter 92, der zwischen der den Strahlteilern 36, 38 des Auskoppelmoduls 32 und den Strahlteilern 40, 42 des Einkoppelmoduls 34 in der Richtung des Doppelpfeils 94 um eine zu den stereoskopischen Teilstrahlengängen 18, 20 in dem Operationsmikroskop parallele Achse 96 geschwenkt werden kann.

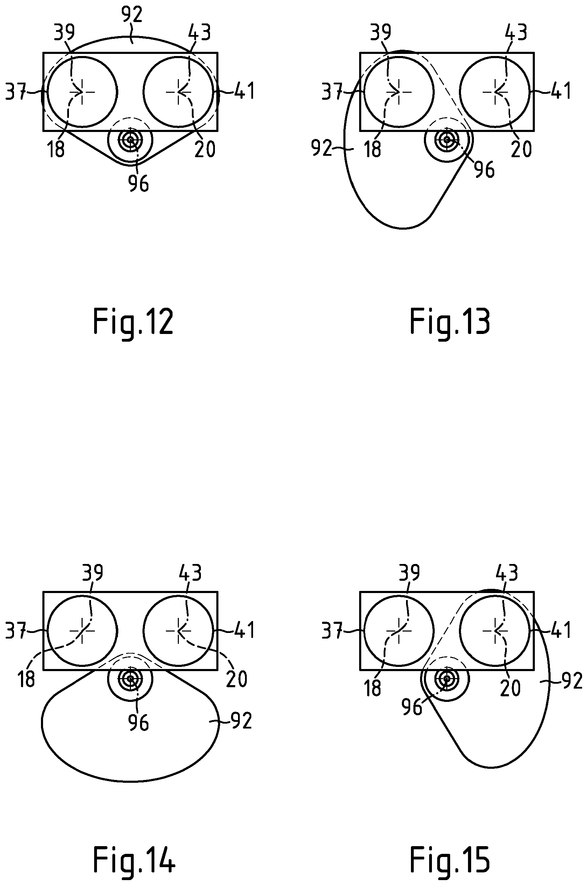

Die Fig. 12 bis Fig. 15 zeigen den Shutter 92 mit dem die optische Achse 39 aufweisenden Beobachtungskanal 37 für den ersten stereoskopischen Teilstrahlengang 18 und dem Beobachtungskanal 41 für den zweiten stereoskopischen Teilstrahlengang 20 und dessen optischer Achse 43. Durch Verschwenken des Shutters 92 um die Achse 96 können bei der Modulbaugruppe die folgenden Einstellungen vorgenommen werden:
In einer ersten, in der Fig. 12 gezeigten Einstellung sind der erste und der zweite stereoskopische Teilstrahlengang 18, 20 gleichzeitig unterbrochen. In der Einstellung der Fig. 13 ist der erste Teilstrahlengang 18 unterbrochen und der zweite stereoskopische Teilstrahlengang 20 freigegeben. Die Fig. 14 zeigt den Shutter in einer Einstellung, in welcher der erste und der zweite stereoskopische Teilstrahlengang 18, 20 von dem Shutter gleichzeitig freigegeben sind. In der Einstellung der Fig. 15 ist der erste stereoskopische Teilstrahlengang 18 freigegeben und der zweite stereoskopische Teilstrahlengang 20 mittels des Shutters 92 unterbrochen.

Die Modulbaugruppe 30' ermöglicht die Anzeige von an der optischen Schnittstelle 44 bereitgestellter Bildinformation in dem ersten und zweiten stereoskopischen Teilstrahlengang 18, 20 in einem Operationsmikroskop wahlweise in Überlagerung zu dem Bild eines Objektbereichs oder ohne dass ein Bild des Objektbereichs zu sehen ist.

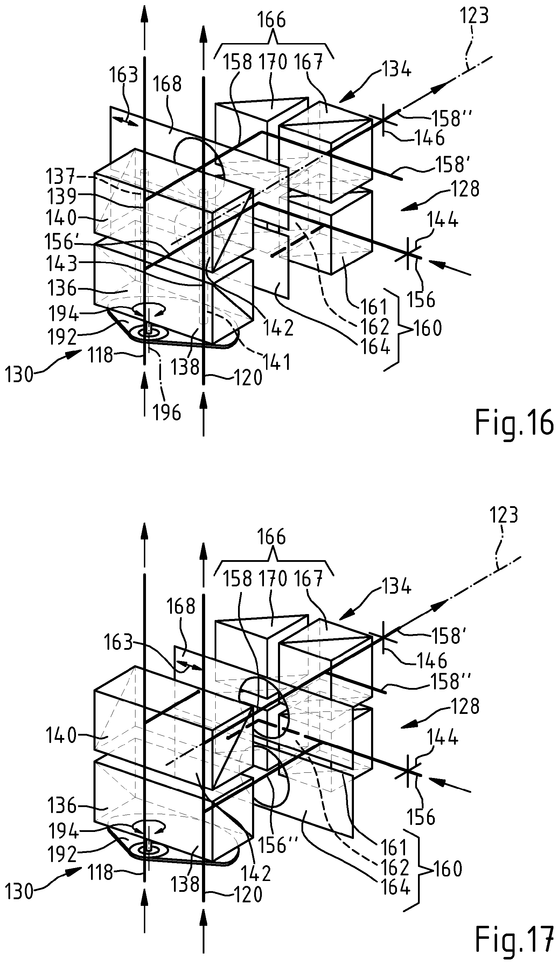

Die Fig. 16 und Fig. 17 zeigen eine weitere Modulbaugruppe 130 für den Einsatz in einem stereoskopischen Operationsmikroskop mit stereoskopischen Teilstrahlengängen 118, 120, einem an einer optischen Schnittstelle 144 zugeführten Strahlengang 156, der als ein Teilstrahlengang 156', 156" in den ersten oder den zweiten stereoskopischen Teilstrahlengang 118, 120 eingekoppelt werden kann, und mit einem an einer optischen Schnittstelle 146 bereitgestellten Strahlengang 158, der aus dem ersten bzw. zweiten stereoskopischen Teilstrahlengang 118, 120 ausgekoppelt ist.

Die Modulbaugruppe 130 ist ein Optikmodul und enthält ein Einkoppelmodul 128. Das Einkoppelmodul 128 weist eine einstellbare Optikbaugruppe 160 auf, die einen als Teilerwürfel ausgebildeten Strahlteiler 161 umfasst, sowie ein als Umlenkprisma ausgebildetes Spiegelelement 162 und eine linearbeweglich angeordnete Lochblende 164 als ein als ein Shutterelement ausgebildetes weiteres optisches Element enthält.

In der Modulbaugruppe 130 gibt es ein Auskoppelmodul 134, das eine einstellbare Optikbaugruppe 166 hat, die ein optisches Element in Form eines Strahlteilers 167 und ein weiteres optisches Element, das ein Shutterelement in Form einer linearbeweglich angeordneten Lochblende 168 ist, sowie ein weiteres als ein Spiegelelement wirkendes Umlenkprisma 170 enthält.

Auf der dem Mikroskop-Hauptobjektiv in einem Operationsmikroskop zugewandten Seite hat die Modulbaugruppe 130 einen Shutter 192, der um die zu der optischen Achse 139, 143 der Beobachtungskanäle 137, 141 parallele Achse 196 in der Richtung des Doppelpfeils 194 gedreht werden kann, wie es vorstehend anhand der Fig. 12 bis Fig. 15 erläutert ist. Damit ist es möglich, den Beobachtungskanal mit dem ersten bzw. zweiten stereoskopischen Teilstrahlengang 118, 120, der den ersten und/oder zweiten Strahlteiler 136, 138, 140, 142 durchsetzt, wahlweise freizugeben oder zu sperren.

Die Fig. 16 zeigt die Modulbaugruppe 130 in einer ersten Einstellung, in welcher der an der optischen Schnittstelle 144 bereitgestellte Strahlengang 156 in den ersten stereoskopischen Teilstrahlengang 118 eingekoppelt ist. Der an der optischen Schnittstelle 146 bereitgestellte Strahlengang ist hier ein aus dem ersten stereoskopischen Teilstrahlengang ausgekoppelter Strahlengang 158.

Die Fig. 17 zeigt die Modulbaugruppe 130 in einer zweiten Einstellung, in welcher der an der optischen Schnittstelle 144 bereitgestellte Strahlengang 156 in den zweiten stereoskopischen Teilstrahlengang 120 eingekoppelt ist und der an der optischen Schnittstelle 146 bereitgestellte Strahlengang ein aus dem zweiten stereoskopischen Teilstrahlengang 120 ausgekoppelter Strahlengang 158 ist.

Ein an der optischen Schnittstelle 144 zugeführter Strahlengang 156 wird in der Modulbaugruppe 130 mittels des Strahlteilers 161 in einen ersten Teilstrahlengang 156' und einen zweiten Teilstrahlengang 156" aufgeteilt. Das Spiegelelement 162 lenkt hier den Teilstrahlengang 156' zu dem in dem ersten stereoskopischen Teilstrahlengang 118 angeordneten Strahlteiler 136. Der Teilstrahlengang 156" ist zu dem in dem zweiten stereoskopischen Teilstrahlengang 120 angeordneten zweiten Strahlteiler 138 geführt. Die Lochblende 164 kann in der Richtung des Doppelpfeils 163 verlagert werden. Damit ist es möglich, in einer ersten Einstellung den ersten Teilstrahlengang 156' in den ersten stereoskopischen Teilstrahlengang 118 einzukoppeln, wobei der zweite Teilstrahlengang 156" des Strahlengangs 156 mittels der Lochblende 164 unterbrochen ist, oder in einer zweiten Einstellung den zweiten Teilstrahlengang 156" in den zweiten stereoskopischen Teilstrahlengang 120 einzukoppeln, wobei dann der erste Teilstrahlengang 156' mittels der Lochblende 164 unterbrochen ist.

In der in der Fig. 16 gezeigten ersten Einstellung der Modulbaugruppe 130 lenkt das Spiegelelement 170 in der einstellbaren Optikbaugruppe 166 des Auskoppelmoduls 134 den mittels des Strahlteilers 140 aus dem ersten stereoskopischen Teilstrahlengang 118 ausgekoppelten Strahlengang 158 zu dem Strahlteiler 167. Der Strahlteiler 167 teilt den Strahlengang 158 in einen ersten und einen zweiten Teilstrahlengang 158', 158". In der in Fig. 16 gezeigten Einstellung der Modulbaugruppe 130 werden die aus dem stereoskopischen Teilstrahlengang 118 ausgekoppelten Lichtstrahlen in dem Teilstrahlengang 158" zu der optischen Schnittstelle 146 gespiegelt.

In der in der Fig. 17 gezeigten zweiten Einstellung der Modulbaugruppe 130 durchsetzen die aus dem zweiten stereoskopischen Teilstrahlengang 120 mittels des Strahlteilers 142 ausgekoppelten Lichtstrahlen die Teilerfläche in dem Strahlteiler 167. Der Strahlteiler 167 teilt den Strahlengang 158 in einen Teilstrahlengang 158', der den Strahlteiler 167 durchsetzt und einen Teilstrahlengang 158', den der Strahlteiler 167 ablenkt. In der in der Fig. 17 gezeigten Einstellung der Modulbaugruppe 130 werden die aus dem stereoskopischen Teilstrahlengang 118 ausgekoppelten Lichtstrahlen, in dem Teilstrahlengang 158' zu der optischen Schnittstelle 146 geführt.

Das Spiegelelement 162 und das Spiegelelement 170 der Modulbaugruppe 130 sind in einem nicht weiter dargestellten Trägerrahmen an einer Justiereinrichtung gehalten, mit der die optische Achse des mittels des Spiegelelements 162, 170 umgelenkten Strahlengangs um zwei zueinander orthogonale Bewegungsachsen justiert werden kann.

Die Modulbaugruppe 130 ermöglicht in einem Operationsmikroskop, dass in den ersten oder in den zweiten stereoskopischen Teilstrahlengang 118, 120 eingespiegelte Bilddateninformation, die an der optischen Schnittstelle 144 bereitgestellt wird, einer Beobachtungsperson wahlweise in Überlagerung zu dem Bild des Objektbereichs zur Anzeige gebracht oder ohne das Bild des Objektbereichs angezeigt werden kann. Dabei steht das von der Beobachtungsperson in dem stereoskopischen Teilstrahlengang wahrnehmbare Bild an der optischen Schnittstelle 146 für eine Kamera zur Verfügung.

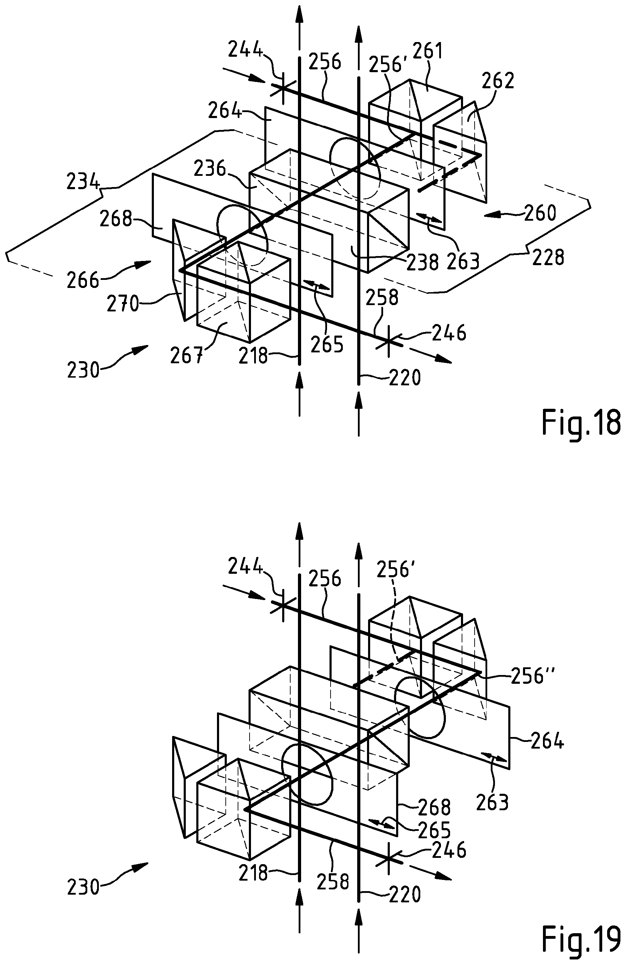

Die Fig. 18, die Fig. 19, die Fig. 20 und die Fig. 21 zeigen eine weitere Modulbaugruppe 230 in unterschiedlichen Einstellungen für den Einsatz in einem stereoskopischen Operationsmikroskop mit stereoskopischen Teilstrahlengängen 218, 220, einem an einer optischen Schnittstelle 244 zugeführten Strahlengang 256 und einem an einer optischen Schnittstelle 246 aus einem stereoskopischen Teilstrahlengang ausgekoppelten Strahlengang 258.

Die Modulbaugruppe 230 hat ein Einkoppelmodul 228 und ein Auskoppelmodul 234 mit einem gemeinsamen Strahlteiler 236 und einem gemeinsamen Strahlteiler 238, die in einem Operationsmikroskop von dem ersten stereoskopischen Teilstrahlengang 218 und von dem zweiten stereoskopischen Teilstrahlgang 220 durchsetzt sind. Die Strahlteiler 236, 238 haben eine Doppelfunktion. Sie dienen für das gleichzeitige Ein- und Auskoppeln eines Strahlengangs in bzw. aus den stereoskopischen Teilstrahlengängen 218, 220.

Das Einkoppelmodul 228 weist eine einstellbare Optikbaugruppe 260 auf, die einen als Teilerwürfel ausgebildeten Strahlteiler 261 umfasst. Die einstellbare Optikbaugruppe 260 enthält weiter ein als Umlenkprisma ausgebildetes Spiegelelement 262 und als ein weiteres optisches Element eine Lochblende 264. Die Lochblende 264 wirkt hier als ein Shutterelement. Sie ist linearbeweglich angeordnet und kann in der Richtung des Doppelpfeils 263 bewegt werden.

Das Auskoppelmodul 234 hat eine einstellbare Optikbaugruppe 266 mit einem optischen Element in Form eines Strahlteilers 267, mit einem weiteren optischen Element in Form einer als Shutterelement wirkenden linearbeweglich angeordneten Lochblende 268 und mit einem weiteren Umlenkprisma 270, das als ein Spiegelelement wirkt.

Ein an der optischen Schnittstelle 244 zugeführter Strahlengang 256 wird mittels des Strahlteilers 261 in einen ersten Teilstrahlengang 256' und einen zweiten Teilstrahlengang 256" aufgeteilt. Das Spiegelelement 262 lenkt den Teilstrahlengang 256" zu dem in dem ersten stereoskopischen Teilstrahlengang 220 angeordneten Strahlteiler 238. Der Teilstrahlengang 256' ist zu dem in dem zweiten stereoskopischen Teilstrahlengang 218 angeordneten zweiten Strahlteiler 236 geführt. Die Lochblende 264 kann in der Richtung des Doppelpfeils 263 verlagert werden. Damit ist es möglich, wahlweise den ersten Teilstrahlengang 256' in den ersten stereoskopischen Teilstrahlengang 218 einzukoppeln, wobei der zweite Teilstrahlengang 256" des Strahlengangs 256 mittels der Lochblende 264 unterbrochen ist, oder den zweiten Teilstrahlengang 256" in den zweiten stereoskopischen Teilstrahlengang 220 einzukoppeln, wobei dann der erste Teilstrahlengang 256' mittels der Lochblende 264 unterbrochen ist.

In der in der Fig. 18 gezeigten Einstellung der Modulbaugruppe 230 wird ein an der optischen Schnittstelle 244 bereitgestellter Strahlengang 256 als ein Strahlengang 256' in den ersten stereoskopischen Teilstrahlengang 218 eingekoppelt. Gleichzeitig wird hier ein aus dem ersten stereoskopischen Teilstrahlengang 218 ausgekoppelter Strahlengang 258 an der optischen Schnittstelle 246 bereitgestellt.

In der Fig. 19 ist die Modulbaugruppe 230 in einer zweiten Einstellung mit einem in den zweiten stereoskopischen Teilstrahlengang 220 eingekoppelten Strahlengang 256" und einem aus diesem stereoskopischen Teilstrahlengang ausgekoppelten Strahlengang 258 gezeigt.

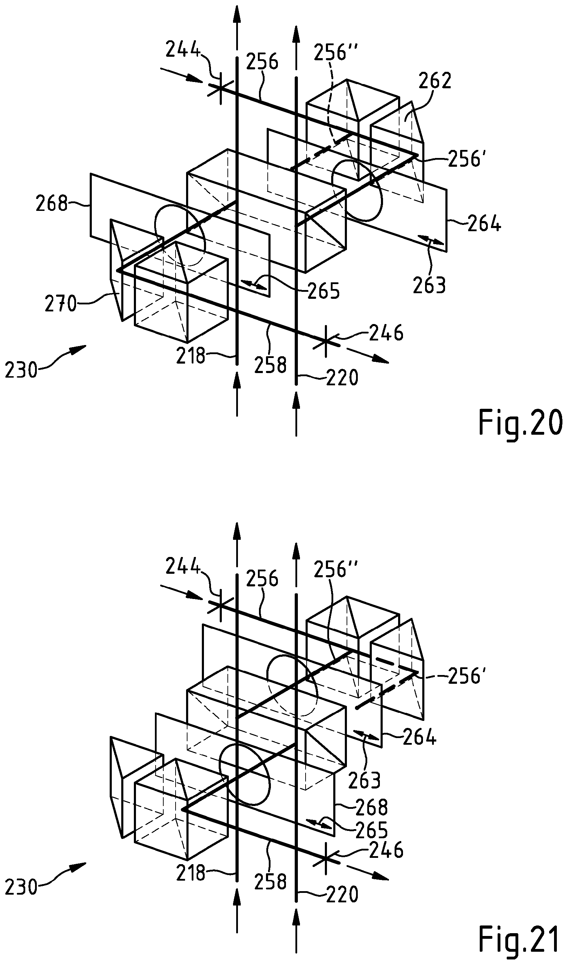

Die Fig. 20 zeigt die Modulbaugruppe 230 in einer dritten Einstellung mit einem in den zweiten stereoskopischen Teilstrahlengang 220 eingekoppelten Strahlengang 256" und einem aus dem ersten stereoskopischen Teilstrahlengang 218 ausgekoppelten Strahlengang 258.

In der Fig. 21 ist die Modulbaugruppe 230 in einer vierten Einstellung mit einem in den ersten stereoskopischen Teilstrahlengang 218 eingekoppelten Strahlengang 256" und einem aus dem zweiten stereoskopischen Teilstrahlengang 220 ausgekoppelten Strahlengang 258 gezeigt.

Das Spiegelelement 262 und das Spiegelelement 270 sind in einem nicht weiter dargestellten Trägerrahmen an einer Justiereinrichtung gehalten, mit der die optische Achse des mittels des Spiegelelements 262, 270 umgelenkten Strahlengangs um zwei zueinander orthogonale Bewegungsachsen justiert werden kann.

Im Vergleich zu den vorstehend erläuterten Modulbaugruppen 30, 30' und 130 minimiert die Modulbaugruppe 230 den Lichtverlust für den ersten und zweiten stereoskopischen Teilstrahlengang 218, 220 in einem Operationsmikroskop, weil jeder stereoskopische Teilstrahlengang 218, 220 hier nur einen Strahlteiler durchsetzt und nicht zwei.

Darüber hinaus hat die Modulbaugruppe 230 eine gegenüber den Modulbaugruppen 30, 30' und 130 verringerte Bauhöhe und ermöglicht deshalb bei einer Integration in Operationsmikroskope besonders kompakte Operationsmikroskop-Bauformen. Zu bemerken ist auch, dass in der Modulbaugruppe 230 die optischen Weglängen in den ersten und zweiten stereoskopischen Teilstrahlengängen 218, 220 minimiert sind.

In einer modifizierten Ausführungsform der Modulbaugruppe 230 kann ein dem drehbeweglichen Shutter 92 in der Modulbaugruppe 30 entsprechender Shutter vorgesehen sein, um damit in einem Operationsmikroskop einen stereoskopischen Teilstrahlengang 218, 220 auf der dem Mikroskop-Hauptobjektiv zugewandten Seite wahlweise freizugeben oder zu sperren. Damit ermöglicht die Modulbaugruppe 230 in einem Operationsmikroskop die Anzeige von an der optischen Schnittstelle 244 bereitgestellter Bildinformation in dem ersten und zweiten stereoskopischen Teilstrahlengang 218, 220 wahlweise in Überlagerung zu dem Bild eines Objektbereichs oder ohne dass ein Bild des Objektbereichs dabei zu sehen ist.

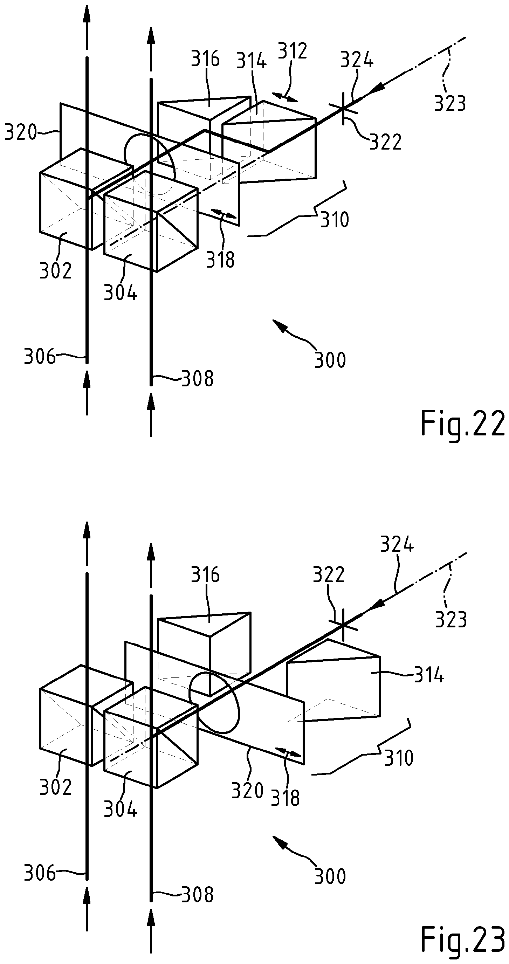

Die Fig. 22 bis Fig. 27 zeigen weitere Einkoppelmodule für ein Operationsmikroskop in unterschiedlichen Einstellungen.

Das in der Fig. 22 gezeigte Einkoppelmodul 300 hat einen ersten Strahlteiler 302 und weist einen zweiten Strahlteiler 304 auf, die in einem stereoskopischen Operationsmikroskop von einem ersten stereoskopischen Teilstrahlengang 306 und einem zweiten stereoskopischen Teilstrahlengang 308 durchsetzt sind. Das Einkoppelmodul 300 hat eine einstellbare Optikbaugruppe 310 mit einem in der Richtung des Doppelpfeils 312 linearbeweglich verlagerbaren Umlenkprisma 314. Die einstellbare Optikbaugruppe 310 enthält weiter ein Umlenkprisma 316, das als Spiegelelement wirkt, und umfasst ein Shutterelement in Form einer in der Richtung des Doppelpfeils 318 linearbeweglich verlagerbaren Lochblende 320.

Die Fig. 22 zeigt das Einkoppelmodul 300 in einer ersten Einstellung. In der in der Fig. 22 gezeigten ersten Einstellung des Einkoppelmoduls 300 wird ein an der optischen Schnittstelle 322 bereitgestellter Strahlengang 324 mittels der Umlenkprismen 314, 316 durch die Lochblende 320 zu dem ersten Strahlteiler 302 geführt und damit in den ersten stereoskopischen Teilstrahlengang 306 eingekoppelt.

Die Fig. 23 zeigt das Einkoppelmodul 300 in einer von der ersten Einstellung verschiedenen zweiten Einstellung. In der zweiten Einstellung des Einkoppelmoduls 300 ist das linearbeweglich verlagerbare Umlenkprisma 314 aus dem Strahlengang 324 bewegt. Die Blendenöffnung der Lochblende 320 befindet sich hier vor dem zweiten Strahlteiler 304, wobei der erste Strahlteiler 302 mittels der Lochblende 320 abgedeckt ist. Der an der optischen Schnittstelle 322 bereitgestellte Strahlengang 324 gelangt hier durch die Blendenöffnung der Lochblende 320 und den zweiten Strahlteiler 304 in den zweiten stereoskopischen Teilstrahlengang 308. Die optische Schnittstelle 322 des Einkoppelmoduls 300 hat hier eine optische Achse 323, die den zweiten Strahlteiler 304 durchsetzt und den zweiten stereoskopischen Teilstrahlengang schneidet.

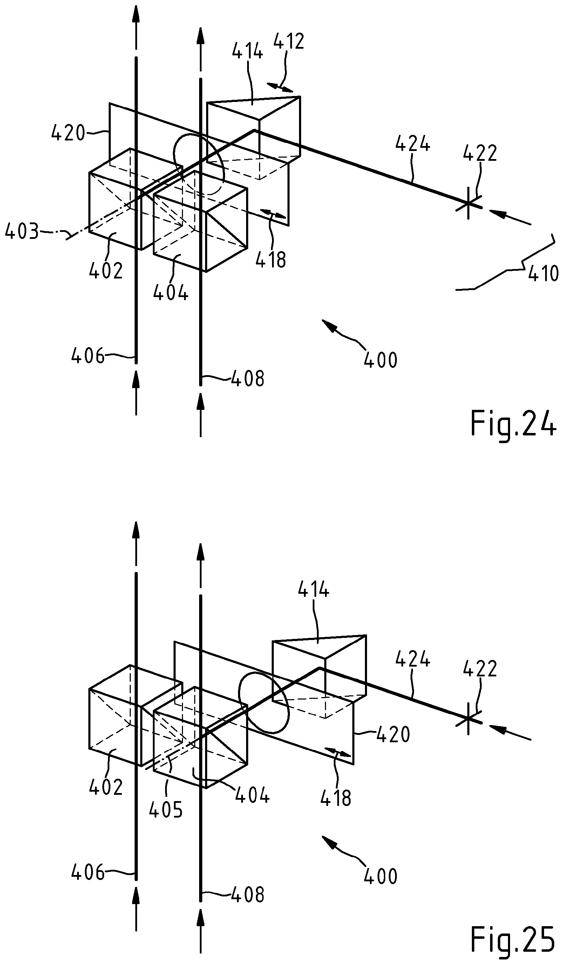

Das in der Fig. 24 gezeigte Einkoppelmodul 400 hat wiederum einen ersten Strahlteiler 402 und weist einen zweiten Strahlteiler 404 auf, die in einem stereoskopischen Operationsmikroskop von einem ersten stereoskopischen Teilstrahlengang 406 und einem zweiten stereoskopischen Teilstrahlengang 408 durchsetzt sind. Das Einkoppelmodul 400 hat eine einstellbare Optikbaugruppe 410 mit einem in der Richtung des Doppelpfeils 412 linearbeweglich verlagerbaren Umlenkprisma 414 und mit einer als Shutterelement wirkenden in der Richtung des Doppelpfeils 418 linearbeweglichen Lochblende 420.

Die Fig. 24 zeigt das Einkoppelmodul 400 in einer ersten Einstellung. In der in der Fig. 24 gezeigten ersten Einstellung des Einkoppelmoduls 400 wird ein an der optischen Schnittstelle 422 bereitgestellter Strahlengang 424 mittels des Umlenkprismas 414 durch die Lochblende 420 in der Richtung der optischen Achse 403 zu dem ersten Strahlteiler 402 geführt und damit in den ersten stereoskopischen Teilstrahlengang 406 eingekoppelt.

Die Fig. 25 zeigt das Einkoppelmodul 400 in einer von der ersten Einstellung verschiedenen zweiten Einstellung. In der zweiten Einstellung des Einkoppelmoduls 400 ist das linearbeweglich verlagerbare Umlenkprisma 414 zusammen mit der Blendenöffnung der Lochblende 420 vor dem zweiten Strahlteiler 404 angeordnet. Die Blendenöffnung der Lochblende 420 befindet sich hier vor dem zweiten Strahlteiler 404. Der an der optischen Schnittstelle 422 bereitgestellte Strahlengang 424 gelangt hier durch die Blendenöffnung der Lochblende 420 und den zweiten Strahlteiler 404 in der Richtung der optischen Achse 405 in den zweiten stereoskopischen Teilstrahlengang 408.

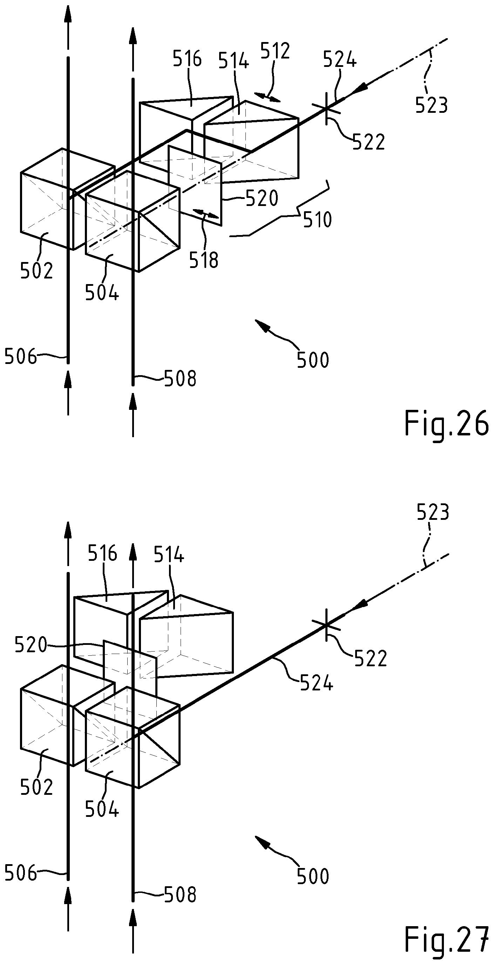

Auch das in der Fig. 26 gezeigte Einkoppelmodul 500 hat einen ersten Strahlteiler 502 und weist einen zweiten Strahlteiler 504 auf, die in einem stereoskopischen Operationsmikroskop von einem ersten stereoskopischen Teilstrahlengang 506 und einem zweiten stereoskopischen Teilstrahlengang 508 durchsetzt sind. Das Einkoppelmodul 500 hat eine einstellbare Optikbaugruppe 510 mit einem ersten und einem zweiten in der Richtung des Doppelpfeils 512 linearbeweglich verlagerbaren Umlenkprisma 514, 516 sowie einem in der Richtung des Doppelpfeils 518 linearbeweglich verlagerbaren Shutter 520.

Die Fig. 26 zeigt das Einkoppelmodul 500 in einer ersten Einstellung. In der in der Fig. 26 gezeigten ersten Einstellung des Einkoppelmoduls 500 wird ein an der optischen Schnittstelle 522 bereitgestellter Strahlengang 524 mittels der Umlenkprismen 514, 516 zu dem ersten Strahlteiler 502 geführt und damit in den ersten stereoskopischen Teilstrahlengang 506 eingekoppelt.

Die Fig. 27 zeigt das Einkoppelmodul 500 in einer von der ersten Einstellung verschiedenen zweiten Einstellung. In der zweiten Einstellung des Einkoppelmoduls 500 sind das linearbeweglich verlagerbare Umlenkprisma 514 und das damit gekoppelte Umlenkprisma 516 aus dem Strahlengang 524 bewegt. Der Shutter 520 befindet sich hier vor dem ersten Strahlteiler 502. Der an der optischen Schnittstelle 522 bereitgestellte Strahlengang 524 gelangt hier direkt zu dem zweiten Strahlteiler 504 und von dort in den zweiten stereoskopischen Teilstrahlengang 508.

Es sei bemerkt, dass in einer modifizierten Ausführungsform für das Einkoppelmodul 500 anstelle der linearbeweglich verlagerbaren Umlenkprismen 514, 516 ein entsprechend linearbewegliches Rhomboidprisma vorgesehen sein kann.

Die optische Schnittstelle 522 des Einkoppelmoduls 500 hat eine optische Achse 523, die den Strahlteiler 504 durchsetzt und die den stereoskopischen Teilstrahlengang 508 schneidet.

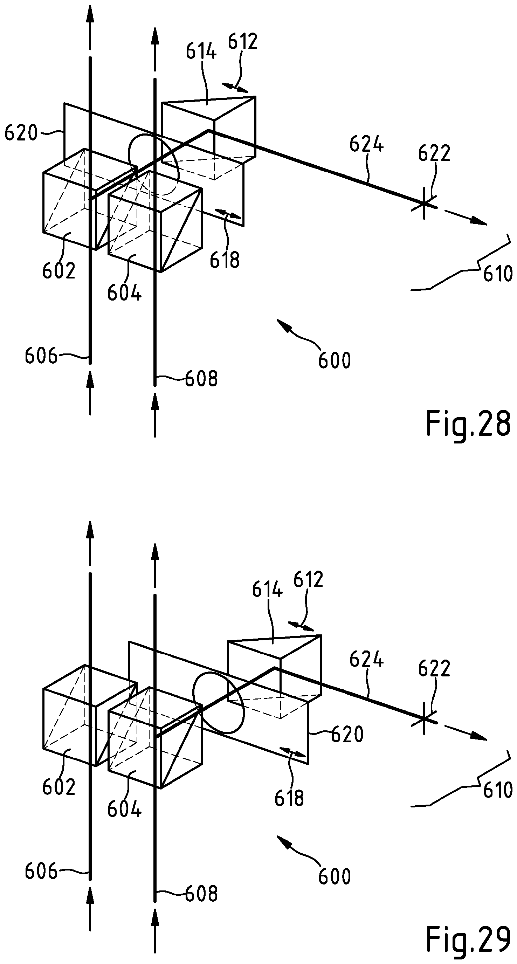

In der Fig. 28, der Fig. 29, der Fig. 30 und der Fig. 31 sind weitere Auskoppelmodule für ein Operationsmikroskop in unterschiedlichen Einstellungen zu sehen.

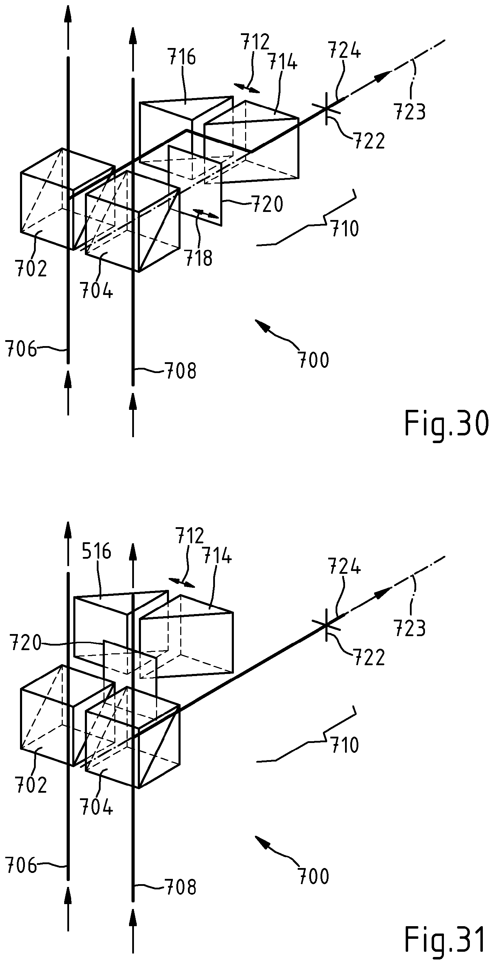

Das in der Fig. 28 und der Fig. 29 gezeigte Auskoppelmodul 600 entspricht in seinem Aufbau und seiner Funktion dem anhand der Fig. 24 und Fig. 25 erläuterten Einkoppelmodul 300. Das Auskoppelmodul 700 in der Fig. 30 und der Fig. 31 entspricht in seinem Aufbau seiner Funktion dem anhand der Fig. 26 und Fig. 27 erläuterten Einkoppelmodul. Baugruppen und Elemente in der Fig. 28 und Fig. 29 bzw. der Fig. 30 und Fig. 31, die zu den Baugruppen und Elementen in den Fig. 24 und Fig. 25 bzw. der Fig. 26 und Fig. 27 identisch sind, haben in der Fig. 28 und Fig. 29 in Bezug auf die Fig. 24 und die Fig. 25 und in der Fig. 30 und der Fig. 31 in Bezug auf die Fig. 26 und die Fig. 27 um die Zahl 200 erhöhte Zahlen als Bezugszeichen. Der aus dem ersten oder zweiten stereoskopischen Teilstrahlengang 606, 608 bzw. 706, 708 ausgekoppelte Strahlengang hat hier das Bezugszeichen 624, 724.

Weitere Abwandlungen und Weiterbildungen von erfindungsgemäßen Einund Auskoppelmodulen können sich auch durch Kombination verschiedener Merkmale der im Vorhergehenden beschriebenen Ausführungsbeispiele ergeben.

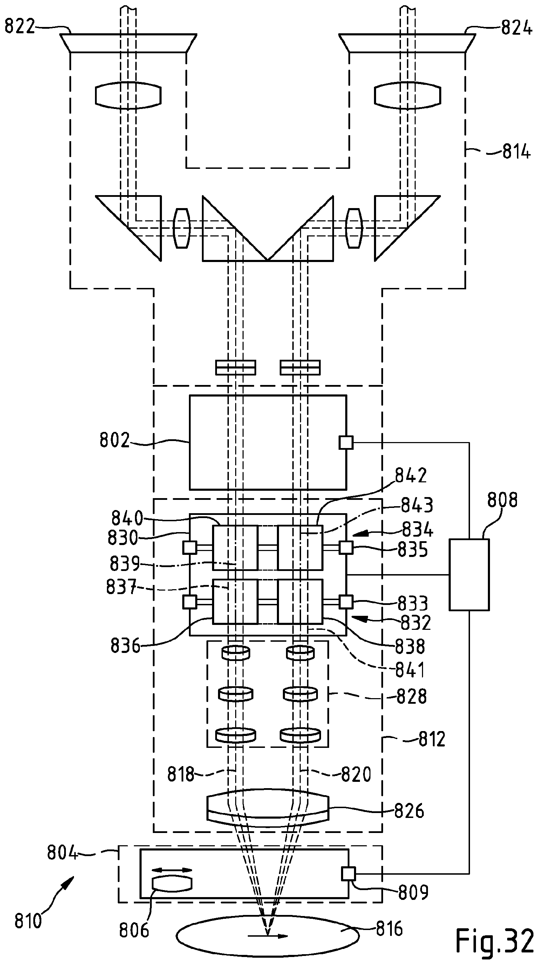

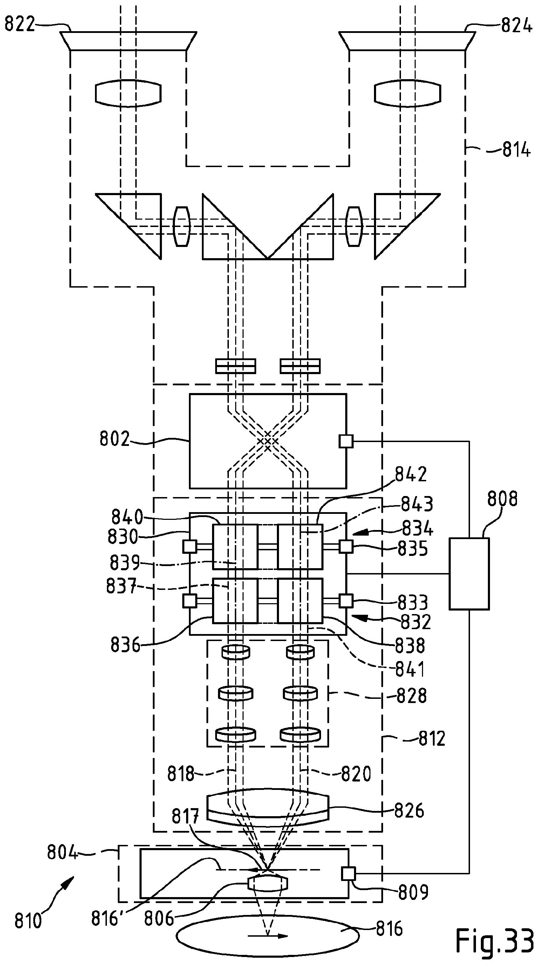

Die Fig. 32 zeigt ein als Ophthalmo-Operationsmikroskop ausgebildetes Operationsmikroskop 810. Soweit die Baugruppen in dem Operationsmikroskop 810 den Baugruppen in dem anhand von Fig. 1 beschriebenen Operationsmikroskop 10 funktional entsprechen, sind diese in der Fig. 32 mit in Bezug auf die Fig. 1 um die Zahl 800 erhöhte Zahlen als Bezugszeichen kenntlich gemacht. Die Modulbaugruppe 830 enthält ein Einkoppelmodul 834 und ein Auskoppelmodul 832, das bevorzugt einen Aufbau hat, wie er sowohl für ein Einkoppelmodul als auch für ein Auskoppelmodul anhand der vorstehenden Figuren beschrieben ist. Das Einkoppelmodul 834 in der Modulbaugruppe 830 dient für das wahlweise Einkoppeln eines Strahlengangs von einem Display in den ersten oder den zweiten stereoskopischen Teilstrahlengang 818, 820. Das Auskoppelmodul 832 in der Modulbaugruppe 830 hat die Aufgabe, wahlweise aus dem ersten oder dem zweiten stereoskopischen Teilstrahlengang 818, 820 einen Strahlengang zu einer Kamera auszukoppeln. Hierzu gibt es in dem Ein- und Auskoppelmodul in der Modulbaugruppe 830 jeweils eine erste und eine zweite einstellbare Optikbaugruppe für die wenigstens zwei unterschiedliche Einstellungen möglich sind. Das Operationsmikroskop 810 enthält darüber hinaus ein System zur Strahlvertauschung und Bildumkehr 802 und umfasst ein Ophthalmoskopie-Vorsatzmodul 804 mit einer Ophthalmoskopierlupe 806. In der Fig. 32 ist das Operationsmikroskop 810 mit aus dem Beobachtungsstrahlengang bewegter Ophthalmoskopierlupe 806 gezeigt. Die Fig. 33 zeigt das Operationsmikroskop 810 mit einer in dem Beobachtungsstrahlengang angeordneten Ophthalmoskopierlupe 806. Die Ophthalmoskopierlupe 806 erzeugt hier ein seitenverkehrtes Bild 817 des Objektbereichs 816 in einer Zwischenbildebene 816', das in dem linken und rechten Okulareinblick 822, 824 von einer Beobachtungsperson mit dem ersten und dem zweiten stereoskopischen Teilstrahlengang 818, 820 stereoskopisch betrachtet werden kann.

In dem Operationsmikroskop 810 gibt es für das Schalten der Modulbaugruppe 830 und des Systems zur Strahlvertauschung und Bildumkehr 802 eine Steuereinrichtung 808. Die Steuereinrichtung wirkt als eine Kopplungseinrichtung, die den Schaltzustand des Systems zur Strahlvertauschung und Bildumkehr 802 mit der Einstellung, d. h. dem Schaltzustand des Ein- und Auskoppelmoduls in der Modulbaugruppe 830 koppelt.

Die Steuereinrichtung 808 ist hier auch mit dem Ophtalmoskopie-Vorsatzmodul 804 verbunden. Mittels eines mit der Steuereinrichtung 808 gesteuerten motorischen Antriebs 809 kann die Ophthalmoskopierlupe 806 wahlweise in den Beobachtungsstrahlengang und aus dem Beobachtungsstrahlengang bewegt werden. Wenn die Ophthalmoskopierlupe 806 in den Beobachtungsstrahlengang bewegt wird, bewirkt die Steuereinrichtung 808 ein Umschalten des Systems zur Strahlvertauschung und Bildumkehr 802 und des Ein- und Auskoppelmoduls 834, 832 in der Modulbaugruppe 830. Dabei wird der Strahlengang von der Displayanzeige in Bezug auf die Einstellung vor dem Umschalten in einen anderen stereoskopischen Teilstrahlengang eingekoppelt und es wird der Kamera in Bezug auf die Einstellung vor dem Umschalten ein anderer stereoskopischer Teilstrahlengang zugeführt.

In einer modifizierten Ausführungsform des Operationsmikroskops 810 kann für das Bewegen der Ophthalmoskopierlupe 806 in dem Ophthalmoskopie-Vorsatzmodul 804 anstelle eines motorischen Antriebs auch ein manueller Antrieb vorgesehen sein. Hier ist es von Vorteil, wenn das Vorsatzmodul 804 einen Positionssensor für das Erfassen der Position der Ophthalmoskopierlupe 806 enthält, der mit der Steuereinrichtung 808 gekoppelt ist, um bei Anordnen der Ophthalmoskopierlupe 806 das Umschalten des Systems zur Strahlvertauschung und Bildumkehr 802 und des Ein- und Auskoppelmoduls 834, 832 in der Modulbaugruppe 830 zu bewirken.

Es sei bemerkt, dass das Operationsmikroskop 810 grundsätzlich auch ohne ein Ophthalmoskopie-Vorsatzmodul 804 mit einer Ophthalmoskopierlupe 806 ausgebildet sein kann. Für das Beobachten des Hintergrunds eines Patientenauges muss dann eben anstelle der Ophthalmoskopierlupe 806 z. B. ein indirektes Kontaktglas eingesetzt werden.

Zusammenfassend ist Folgendes festzuhalten: Die Erfindung betrifft ein Einkoppelmodul 128 für das wahlweise Einkoppeln eines Strahlengangs 156 in einen ersten oder zweiten stereoskopischen Teilstrahlengang 118, 120 in einem Operationsmikroskop und mit einer optischen Schnittstelle 144 für das Zuführen eines einzukoppelnden Strahlengangs 156. Das Einkoppelmodul 128 hat einen in dem ersten stereoskopischen Teilstrahlengang 118 anordenbaren ersten Strahlteiler 136 und einen in dem zweiten stereoskopischen Teilstrahlengang 120 anordenbaren zweiten Strahlteiler 138. Das Einkoppelmodul 128 enthält eine einstellbare Optikbaugruppe 160, die wahlweise einen an der optischen Schnittstelle 144 bereitgestellten Strahlengang 156 für das Einkoppeln in den ersten oder zweiten stereoskopischen Teilstrahlengang 118, 120 dem ersten Strahlteiler 136 oder dem zweiten Strahlteiler 138 zuführt. Erfindungsgemäß umfasst die einstellbare Optikbaugruppe 160 für das Umschalten des Strahlengangs mindestens ein aus einer ersten Position in eine zweite Position und umgekehrt linearbeweglich verlagerbares optisches Element 164, das in der ersten Position und/oder der zweiten Position in dem einzukoppelnden Strahlengang 156 angeordnet ist. Die Erfindung betrifft außerdem ein Auskoppelmodul 134 für das wahlweise Auskoppeln eines Strahlengangs aus einem ersten oder zweiten stereoskopischen Teilstrahlengang 118, 120 in einem Operationsmikroskop, das eine optische Schnittstelle 146 für das Bereitstellen des Strahlengangs, einen in dem ersten Teilstrahlengang 118 anordenbaren ersten Strahlteiler 140 und einen in dem zweiten Teilstrahlengang anordenbaren zweiten Strahlteiler 142 aufweist. Das Auskoppelmodul 134 enthält außerdem eine einstellbare Optikbaugruppe 166, die der optischen Schnittstelle 146 wahlweise einen aus dem ersten oder dem zweiten stereoskopischen Teilstrahlengang ausgekoppelten Strahlengang 158 zuführt. Erfindungsgemäß umfasst die einstellbare Optikbaugruppe 166 für das Umschalten des Strahlengangs 158 mindestens ein von einer ersten Position in eine zweite Position linearbeweglich verlagerbares optisches Element 168, das in der ersten Position und/oder der zweiten Position in dem ausgekoppelten Strahlengang 158 angeordnet ist.

### Bezugszeichenliste

- 10: Operationsmikroskop
- 12: Grundkörper
- 14: Binokulartubus
- 16: Objektbereich
- 18,20: Teilstrahlengang
- 22: linker Okulareinblick
- 23: optische Achse
- 24: rechter Okulareinblick
- 26: Hauptobjektiv
- 28: Vergrößerungssystem
- 30, 30': Modulbaugruppe
- 32: Auskoppelmodul
- 33,35: Trägerrahmen
- 34: Einkoppelmodul
- 36, 38, 40, 42: Strahlteiler
- 37,41: Beobachtungskanäle
- 39, 43, 45, 47: optische Achse
- 44,46: Schnittstellen
- 50: Displaymodul
- 54: Kamera
- 56, 56', 56", 58: Strahlengang
- 60,66: Optikbaugruppe
- 61: Strahlteiler
- 62: Spiegelelement
- 63: Doppelpfeil
- 64, 68: Lochblende
- 67, 70: Spiegelelement, Umlenkprisma
- 72: Justiereinrichtung
- 74: Halter
- 76: Befestigungsschraube
- 78: Träger
- 80, 82: Justierschrauben
- 83: Madenschraube
- 84,86: Bewegungsachsen
- 88, 90,: Doppelpfeile
- 92: Shutter
- 94: Doppelpfeil
- 96: parallele Achse

- 118, 120: Teilstrahlengang
- 123: optische Achse
- 128: Einkoppelmodul
- 130: Modulbaugruppe
- 134: Auskoppelmodul
- 136, 138, 140, 142: Strahlteiler
- 137, 141: Beobachtungskanäle
- 139, 143: optische Achsen
- 144, 146: optische Schnittstellen
- 156, 156', 156": Teilstrahlengang, Strahlengang
- 158, 158', 158": Teilstrahlengang, Strahlengang
- 160, 166: Optikbaugruppe
- 161, 167: Strahlteiler
- 162,: Spiegelelement
- 163: Doppelpfeil
- 164, 168: Lochblende
- 170: Spiegelelement, Umlenkprisma
- 192: Shutter
- 194: Doppelpfeil
- 196: parallele Achse

- 218, 220: Teilstrahlengang
- 228: Einkoppelmodul
- 230: Modulbaugruppe
- 234: Auskoppelmodul
- 236, 238: Strahlteiler
- 244, 246: Schnittstellen
- 256, 256', 256", 258: Teilstrahlengang, Strahlengang
- 260: Optikbaugruppe
- 261, 267: Strahlteiler
- 262: Spiegelelement
- 263, 265: Doppelpfeil
- 264,268: Lochblende
- 266: Optikbaugruppe
- 270: Spiegelelement, Umlenkprisma

- 300: Einkoppelmodul
- 302, 304: Strahlteiler
- 306, 308: Teilstrahlengang
- 310: Optikbaugruppe
- 312, 318: Doppelpfeil
- 314, 316: Spiegelelement, Umlenkprisma
- 320: Lochblende
- 322: optische Schnittstellen
- 323: optische Achse
- 324: Strahlengang

- 400: Einkoppelmodul
- 402, 404: Strahlteiler
- 403, 405: optische Achse
- 406, 408: Teilstrahlengang
- 410: Optikbaugruppe
- 412: Doppelpfeil
- 414: Spiegelelement, Umlenkprisma
- 418: Doppelpfeil
- 420: Lochblende
- 422: optische Schnittstellen
- 424: Strahlengang

- 500: Einkoppelmodul
- 502, 504: Strahlteiler
- 506, 508: Teilstrahlengang
- 510: Optikbaugruppe
- 512, 518: Doppelpfeil
- 514, 516: Spiegelelement, Umlenkprisma
- 520: Shutter
- 522: optische Schnittstellen
- 523: optische Achse
- 524: Strahlengang

- 600: Auskoppelmodul
- 602, 604: Strahlteiler
- 606,608: Teilstrahlengang
- 610: Optikbaugruppe
- 614: Spiegelelement, Umlenkprisma
- 612, 618: Doppelpfeil
- 620: Lochblende
- 622: Schnittstellen
- 624: Strahlengang

- 700: Auskoppelmodul
- 702, 704: Strahlteiler
- 706, 708: Teilstrahlengang
- 710: Optikbaugruppe
- 712, 718: Doppelpfeil
- 714, 716: Spiegelelement, Umlenkprisma
- 720: Shutter
- 722: optische Schnittstellen
- 723: optische Achse
- 724: Strahlengang

- 802: System zur Strahlvertauschung und Bildumkehr
- 804: Ophthalmoskopie-Vorsatzmodul
- 806: Ophthalmoskopierlupe
- 808: Steuereinrichtung
- 809: Antrieb
- 810: Operationsmikroskop
- 812: Grundkörper
- 814: Binokulartubus
- 816: Objektbereich
- 816': Zwischenbildebene
- 817: Bild
- 818, 820: Teilstrahlengang
- 822: linker Okulareinblick
- 824: rechter Okulareinblick
- 826: Hauptobjektiv
- 828: Zoomsystem, Vergrößerungssystem
- 830: Modulbaugruppe
- 832: Auskoppelmodul
- 833,835: Trägerrahmen
- 834: Einkoppelmodul
- 836, 838, 840, 842: Strahlteiler
- 837, 841: Beobachtungskanäle
- 839, 843: optische Achsen

## Patentansprüche

1. Einkoppelmodul (300, 500) für das wahlweise Einkoppeln eines an einer ortsfesten optischen Schnittstelle (322, 522) bereitgestellten Strahlengangs (324, 524) in einen ersten oder zweiten stereoskopischen Teilstrahlengang (306, 308, 506, 508) in einem optischen Instrument (10),
mit einem in dem ersten stereoskopischen Teilstrahlengang (306, 506) angeordneten ersten Strahlteiler (302, 502),
mit einem in dem zweiten stereoskopischen Teilstrahlengang (308, 508) angeordneten zweiten Strahlteiler (304, 504),
mit einer einstellbaren Optikbaugruppe (310, 510), die den an der optischen Schnittstelle (322, 522) bereitgestellten Strahlengang (324, 524) wahlweise dem ersten Strahlteiler (302, 502) oder dem zweiten Strahlteiler (304, 504) zuführt,
**dadurch gekennzeichnet, dass**
die einstellbare Optikbaugruppe (310, 510) mindestens ein aus einer ersten Position in eine zweite Position und umgekehrt relativ zu der optischen Schnittstelle (322, 522) linearbeweglich verlagerbares Spiegelelement (314, 514) enthält, das in der ersten Position in dem an der optischen Schnittstelle (322, 522) bereitgestellten Strahlengang (324, 524) angeordnet ist, wobei
das linearbeweglich verlagerbare Spiegelelement (314, 514) in der ersten Position den an der optischen Schnittstelle (322, 522) bereitgestellten Strahlengang (324, 524) zu einem weiteren Spiegelelement (316, 516) lenkt, das den Strahlengang (324) dem ersten Strahlteiler (302, 502) für das Einkoppeln in den ersten stereoskopischen Teilstrahlengang (306, 506) zuführt, und in der zweiten Position den an der optischen Schnittstelle bereitgestellten Strahlengang (324, 524) für das Einkoppeln in den zweiten stereoskopischen Teilstrahlengang (308, 508) zu dem zweiten Strahlteiler (304, 504) freigibt, wobei
die optische Achse (323, 523) des an der optischen Schnittstelle (322, 522) bereitgestellten Strahlengangs (324, 524) eine den zweiten Strahlteiler (304, 504) durchsetzende Symmetrieachse ist; und wobei
die einstellbare Optikbaugruppe (310, 510) ein mit dem linearbeweglich verlagerbaren Spiegelelement (314, 514) bewegungsgekoppeltes linearbeweglich verlagerbares Shutterelement (320, 520) für das wahlweise Abdecken des ersten Strahlteilers (302, 502) und Freigeben des zweiten Strahlteilers (304, 504) oder das Freigeben des ersten Strahlteilers (302, 502) und Abdecken des zweiten Strahlteilers (304, 504) enthält.

2. Auskoppelmodul (32, 700) für das wahlweise Auskoppeln eines Strahlengangs (58, 724) zu einer ortsfesten optischen Schnittstelle (46, 722) aus einem ersten oder zweiten stereoskopischen Teilstrahlengang (18, 20, 706, 708) in einem optischen Instrument (10),
mit einem in dem ersten stereoskopischen Teilstrahlengang (18, 708) angeordneten ersten Strahlteiler (36, 704),
mit einem in dem zweiten stereoskopischen Teilstrahlengang (20, 706) angeordneten zweiten Strahlteiler (38, 702),
mit einer einstellbaren Optikbaugruppe (66, 710), die den ausgekoppelten Strahlengang (58, 724) zu der optischen Schnittstelle (46, 722) wahlweise von dem ersten Strahlteiler (36, 704) oder dem zweiten Strahlteiler (38, 702) zuführt,
**dadurch gekennzeichnet, dass**
die einstellbare Optikbaugruppe (66, 710) mindestens ein aus einer ersten Position in eine zweite Position und umgekehrt relativ zu der optischen Schnittstelle (46, 722) linearbeweglich verlagerbares Spiegelelement (67, 714) enthält, das in der ersten Position in dem der optischen Schnittstelle (46, 722) zugeführten Strahlengang (58, 724) angeordnet ist, wobei
das linearbeweglich verlagerbare Spiegelelement (67, 714) in der ersten Position den der Schnittstelle (46, 722) zugeführten Strahlengang (58, 724) von einem weiteren Spiegelelement (70, 716) erhält, dem der Strahlengang (58, 724) von dem zweiten Strahlteiler (38, 702) für das Auskoppeln aus dem zweiten stereoskopischen Teilstrahlengang (20, 706) zugeführt wird, und in der zweiten Position den der Schnittstelle (46, 722) zugeführten Strahlengang (58, 724) aus dem zweiten stereoskopischen Teilstrahlengang (308, 706) zu der optischen Schnittstelle (722) freigibt, wobei
an der optischen Schnittstelle (46, 722) die optische Achse (23, 723) des der optischen Schnittstelle (46, 722) zugeführten Strahlengangs (58, 724) eine den ersten Strahlteiler (36, 704) durchsetzende Symmetrieachse ist; und wobei
die einstellbare Optikbaugruppe (66, 710) ein mit dem linearbeweglich verlagerbaren Spiegelelement (67, 714) bewegungsgekoppeltes linearbeweglich verlagerbares Shutterelement (64, 720) für das wahlweise Abdecken des ersten Strahlteilers (36, 704) und Freigeben des zweiten Strahlteilers (38, 702) oder das Freigeben des ersten Strahlteilers (36, 704) und Abdecken des zweiten Strahlteilers (38, 702) enthält.

3. Modul nach Anspruch 1 oder 2, **dadurch gekennzeichnet, dass** das weitere Spiegelelement (70, 716) zu der optischen Schnittstelle (46, 322) ortsfest ist.

4. Modul nach Anspruch 1 oder 2, **dadurch gekennzeichnet, dass** das weitere Spiegelelement (716) zu der optischen Schnittstelle (722) linearbeweglich verlagerbar ist.

5. Einkoppelmodul (400) für das wahlweise Einkoppeln eines an einer ortsfesten optischen Schnittstelle (422) bereitgestellten Strahlengangs (424) in einen ersten oder zweiten stereoskopischen Teilstrahlengang (406, 408) in einem optischen Instrument (10),
mit einem in dem ersten stereoskopischen Teilstrahlengang (406) angeordneten ersten Strahlteiler (402),
mit einem in dem zweiten stereoskopischen Teilstrahlengang (408) angeordneten zweiten Strahlteiler (404),
mit einer einstellbaren Optikbaugruppe (410), die den an der optischen Schnittstelle (422) bereitgestellten Strahlengang (424) wahlweise dem ersten Strahlteiler (402) oder dem zweiten Strahlteiler (404) zuführt,
**dadurch gekennzeichnet, dass**
die einstellbare Optikbaugruppe (410) mindestens ein aus einer ersten Position in eine zweite Position und umgekehrt relativ zu der optischen Schnittstelle (422) linearbeweglich verlagerbares Spiegelelement (414) enthält, das in der ersten Position in dem an der optischen Schnittstelle (422) bereitgestellten Strahlengang (424) angeordnet ist und den bereitgestellten Strahlengang (424) dem ersten Strahlteiler (402) für das Einkoppeln in den ersten stereoskopischen Teilstrahlengang (406) zuführt, wobei
das linearbeweglich verlagerbare Spiegelelement (414) auch in der zweiten Position in dem bereitgestellten einzukoppelnden Strahlengang (424) angeordnet ist und den Strahlengang dem zweiten Strahlteiler (404) für das Einkoppeln in den zweiten stereoskopischen Teilstrahlengang (408) zuführt; und wobei
die einstellbare Optikbaugruppe (410) ein mit dem linearbeweglich verlagerbaren Spiegelelement (414) bewegungsgekoppeltes linearbeweglich verlagerbares Shutterelement (420) für das wahlweise Abdecken des ersten Strahlteilers (402) und Freigeben des zweiten Strahlteilers (404) oder das Freigeben des ersten Strahlteilers (402) und Abdecken des zweiten Strahlteilers (404) enthält.

6. Auskoppelmodul (600) für das wahlweise Auskoppeln eines Strahlengangs (624) zu einer ortsfesten optischen Schnittstelle (622) aus einem ersten oder zweiten stereoskopischen Teilstrahlengang (606, 608) in einem optischen Instrument (10),
mit einem in dem ersten stereoskopischen Teilstrahlengang (606) angeordneten ersten Strahlteiler (602),
mit einem in dem zweiten stereoskopischen Teilstrahlengang (608) angeordneten zweiten Strahlteiler (604),
mit einer einstellbaren Optikbaugruppe (610), die der optischen Schnittstelle (622) wahlweise einen aus dem ersten oder dem zweiten stereoskopischen Teilstrahlengang (606, 608) ausgekoppelten Strahlengang zuführt,
**dadurch gekennzeichnet, dass**
die einstellbare Optikbaugruppe (610) mindestens ein aus einer ersten Position in eine zweite Position und umgekehrt relativ zu der optischen Schnittstelle (622) linearbeweglich verlagerbares Spiegelelement (614) enthält, das in der ersten Position in dem auszukoppelnden Strahlengang (624) angeordnet ist und den Strahlengang von dem ersten Strahlteiler (602) für das Auskoppeln aus dem ersten stereoskopischen Teilstrahlengang (606) erhält, wobei
das linearbeweglich verlagerbare Spiegelelement (614) auch in der zweiten Position in dem auszukoppelnden Strahlengang (624) angeordnet ist und den Strahlengang von dem zweiten Strahlteiler (604) für das Auskoppeln aus dem zweiten stereoskopischen Teilstrahlengang (608) erhält; und wobei
die einstellbare Optikbaugruppe (610) ein mit dem linearbeweglich verlagerbaren Spiegelelement (614) bewegungsgekoppeltes linearbeweglich verlagerbares Shutterelement (620) für das wahlweise Abdecken des ersten Strahlteilers (602) und Freigeben des zweiten Strahlteilers (604) oder das Freigeben des ersten Strahlteilers (602) und Abdecken des zweiten Strahlteilers (604) enthält.

7. Modul nach einem der Ansprüche 1 bis 4, **gekennzeichnet durch** einen die Optikbaugruppe (60) aufnehmenden Trägerrahmen (33), in dem das weitere Spiegelelement (316) an einer Justiereinrichtung (72) gehalten ist, mit der das weitere Spiegelelement (316) für das Einstellen der optischen Achse (45) des dem ersten Strahlteiler (40) zugeführten Strahlengangs (56') justiert werden kann.

8. Modul nach einem der Ansprüche 1 bis 7, **gekennzeichnet durch** einen die Optikbaugruppe (410) aufnehmenden Trägerrahmen (33), in dem das Spiegelelement (414) an einer Justiereinrichtung (72) gehalten ist, mit der das Spiegelelement (414) für das Einstellen der optischen Achse des dem ersten Strahlteiler (402) und dem zweiten Strahlteiler (404) zugeführten Strahlengangs justiert werden kann.

9. Operationsmikroskop (10) für das stereoskopische Beobachten eines Objektbereichs (16) mit einem ersten (18) und mit einem zweiten (20) stereoskopischen Teilstrahlengang und mit einem gemäß einem der Ansprüche 1, 3 bis 5, 7 oder 8 ausgebildeten Einkoppelmodul (34, 128, 228, 300, 400, 500) und/oder mit einem gemäß einem der Ansprüche 2, 3, 4 oder 6 bis 8 ausgebildeten Auskoppelmodul (32, 134, 234, 600, 700).

## Claims

1. Input coupling module (300, 500) for selectively coupling a beam path (324, 524) provided at a stationary optical interface (322, 522) into a first or second stereoscopic partial beam path (306, 308, 506, 508) in an optical instrument (10),
with a first beam splitter (302, 502) arranged in the first stereoscopic partial beam path (306, 506),
with a second beam splitter (304, 504) arranged in the second stereoscopic partial beam path (308, 508) ,
with an adjustable optics assembly (310, 510) which selectively guides the beam path (324, 524) provided at the optical interface (322, 522) to the first beam splitter (302, 502) or the second beam splitter (304, 504),
**characterized in that**
the adjustable optics assembly (310, 510) contains at least one mirror element (314, 514) displaceable by linear movement from a first position to a second position, and vice versa, relative to the optical interface (322, 522), said mirror element being arranged in the beam path (324, 524) provided at the optical interface (322, 522) in the first position,
wherein
the mirror element (314, 514) displaceable by linear movement, in the first position, directs the beam path (324, 524) provided at the optical interface (322, 522) to a further mirror element (316, 516) guiding the beam path (324) to the first beam splitter (302, 502) for coupling into the first stereoscopic partial beam path (306, 506), and, in the second position, uncovers the beam path (324, 524) provided at the optical interface for coupling into the second stereoscopic partial beam path (308, 508) to the second beam splitter (304, 504), wherein
the optical axis (323, 523) of the beam path (324, 524) provided at the optical interface (322, 522) is an axis of symmetry passing through the second beam splitter (304, 504); and wherein
the adjustable optics assembly (310, 510) contains a shutter element (320, 520) displaceable by linear movement and coupled in terms of movement with the mirror element (314, 514) displaceable by linear movement, for selectively covering the first beam splitter (302, 502) and uncovering the second beam splitter (304, 504) or uncovering the first beam splitter (302, 502) and covering the second beam splitter (304, 504).

2. Output coupling module (32, 700) for selectively decoupling a beam path (58, 724) to a stationary optical interface (46, 722) from a first or second stereoscopic partial beam path (18, 20, 706, 708) in an optical instrument (10),
with a first beam splitter (36, 704) arranged in the first stereoscopic partial beam path (18, 708),
with a second beam splitter (38, 702) arranged in the second stereoscopic partial beam path (20, 706),
with an adjustable optics assembly (66, 710) which guides the decoupled beam path (58, 724) to the optical interface (46, 722) selectively from the first beam splitter (36, 704) or the second beam splitter (38, 702),
**characterized in that**
the adjustable optics assembly (66, 710) contains at least one mirror element (67, 714) displaceable by linear movement from a first position to a second position, and vice versa, relative to the optical interface (46, 722), said mirror element being arranged in the beam path (58, 724) guided to the optical interface (46, 722) in the first position,
wherein
the mirror element (67, 714) displaceable by linear movement, in the first position, receives the beam path (58, 724) guided to the interface (46, 722) from a further mirror element (70, 716), to which the beam path (58, 724) from the second beam splitter (38, 702) for decoupling from the second stereoscopic partial beam path (20, 706) is guided, and, in the second position, uncovers the beam path (58, 724) guided to the interface (46, 722) from the second stereoscopic partial beam path (308, 706) to the optical interface (722), wherein
at the optical interface (46, 722), the optical axis (23, 723) of the beam path (58, 724) guided to the optical interface (46, 722) is an axis of symmetry passing through the first beam splitter (36, 704); and wherein
the adjustable optics assembly (66, 710) contains a shutter element (64, 720) displaceable by linear movement and coupled in terms of movement with the mirror element (67, 714) displaceable by linear movement, for selectively covering the first beam splitter (36, 704) and uncovering the second beam splitter (38, 702) or uncovering the first beam splitter (36, 704) and covering the second beam splitter (38, 702).

3. Module according to Claim 1 or 2, **characterized in that** the further mirror element (70, 716) on the path to the optical interface (46, 322) is stationary.

4. Module according to Claim 1 or 2, **characterized in that** the further mirror element (716) on the path to the optical interface (722) is displaceable by linear movement.

5. Input coupling module (400) for selectively coupling a beam path (424) provided at a stationary optical interface (422) into a first or second stereoscopic partial beam path (406, 408) in an optical instrument (10),
with a first beam splitter (402) arranged in the first stereoscopic partial beam path (406),
with a second beam splitter (404) arranged in the second stereoscopic partial beam path (408),
with an adjustable optics assembly (410) which selectively guides the beam path (424) provided at the optical interface (422) to the first beam splitter (402) or the second beam splitter (404),
**characterized in that**
the adjustable optics assembly (410) contains at least one mirror element (414) displaceable by linear movement from a first position to a second position, and vice versa, relative to the optical interface (422), said mirror element being arranged in the beam path (424) provided at the optical interface (422) in the first position and guiding the provided beam path (424) to the first beam splitter (402) for coupling into the first stereoscopic partial beam path (406), wherein
the mirror element (414) displaceable by linear movement is also arranged in the provided beam path (424) to be coupled-in in the second position and it guides the beam path to the second beam splitter (404) for coupling into the second stereoscopic partial beam path (408); and wherein
the adjustable optics assembly (410) contains a shutter element (420) displaceable by linear movement and coupled in terms of movement with the mirror element (414) displaceable by linear movement, for selectively covering the first beam splitter (402) and uncovering the second beam splitter (404) or uncovering the first beam splitter (402) and covering the second beam splitter (404).

6. Output coupling module (600) for selectively decoupling a beam path (624) to a stationary optical interface (622) from a first or second stereoscopic partial beam path (606, 608) in an optical instrument (10),
with a first beam splitter (602) arranged in the first stereoscopic partial beam path (606),
with a second beam splitter (604) arranged in the second stereoscopic partial beam path (608),
with an adjustable optics assembly (610) which selectively guides a beam path decoupled from the first or the second stereoscopic partial beam path (606, 608) to the optical interface (622),
**characterized in that**
the adjustable optics assembly (610) contains at least one mirror element (614), which is displaceable by linear movement from a first position to a second position, and vice versa, relative to the optical interface (622), said mirror element being arranged in the beam path (624) to be decoupled in the first position and receiving the beam path from the first beam splitter (602) for decoupling from the first stereoscopic partial beam path (606), wherein
the mirror element (614) displaceable by linear movement is also arranged in the beam path (624) to be decoupled in the second position and it receives the beam path from the second beam splitter (604) for decoupling from the second stereoscopic partial beam path (608); and wherein
the adjustable optics assembly (610) contains a shutter element (620) displaceable by linear movement and coupled in terms of movement with the mirror element (614) displaceable by linear movement, for selectively covering the first beam splitter (602) and uncovering the second beam splitter (604) or uncovering the first beam splitter (602) and covering the second beam splitter (604).

7. Module according to any one of Claims 1 to 4, **characterized by** a support frame (33) receiving the optics assembly (60), in which support frame the further mirror element (316) is held at an adjustment device (72), by means of which the further mirror element (316) can be adjusted for setting the optical axis (45) of the beam path (56') guided to the first beam splitter (40).

8. Module according to any one of claims 1 to 7, **characterized by** a support frame (33) receiving the optics assembly (410), in which support frame the mirror element (414) is held at an adjustment device (72), by means of which the mirror element (414) can be adjusted for setting the optical axis of the beam path guided to the first beam splitter (402) and the second beam splitter (404).

9. Surgical microscope (10) for the stereoscopic observation of an object region (16), with a first (18) and with a second (20) stereoscopic partial beam path and with an input coupling module (34, 128, 228, 300, 400, 500) embodied according to any one of Claims 1, 3 to 5, 7 or 8 and/or with an output coupling module (32, 134, 234, 600, 700) embodied according to any one of Claims 2, 3, 4 or 6 to 8.

## Revendications

1. Module d'injection (300, 500) pour l'injection sélective d'un trajet de faisceau (324, 524) mis à disposition au niveau d'une interface optique (322, 522) en position fixe dans un premier ou un deuxième trajet de faisceau partiel (306, 308, 506, 508) stéréoscopique dans un instrument optique (10),
comprenant un premier diviseur de faisceau (302, 502) disposé dans le premier trajet de faisceau partiel (306, 506) stéréoscopique,
comprenant un deuxième diviseur de faisceau (304, 504) disposé dans le deuxième trajet de faisceau partiel (308, 508) stéréoscopique,
comprenant un sous-ensemble optique (310, 510) réglable qui achemine le trajet de faisceau (324, 524) mis à disposition au niveau de l'interface optique (322, 522) sélectivement au premier diviseur de faisceau (302, 502) ou au deuxième diviseur de faisceau (304, 504), **caractérisé en ce que**
le sous-ensemble optique (310, 510) réglable contient au moins un élément miroir (314, 514) qui peut être positionné en mobilité linéaire d'une première position dans une deuxième position et inversement par rapport à l'interface optique (322, 522) et qui, dans la première position, est disposé dans le trajet de faisceau (324, 524) mis à disposition au niveau de l'interface optique (322, 522),
dans la première position, l'élément miroir (314, 514) qui peut être positionné en mobilité linéaire déviant le trajet de faisceau (324, 524) mis à disposition au niveau de l'interface optique (322, 522) vers un élément miroir supplémentaire (316, 516), qui achemine le trajet de faisceau (324) au premier diviseur de faisceau (302, 502) pour l'injection dans le premier trajet de faisceau partiel (306, 506) stéréoscopique et, dans la deuxième position, libérant le trajet de faisceau (324, 524) mis à disposition au niveau de l'interface optique vers le deuxième diviseur de faisceau (304, 504) pour l'injection dans le deuxième trajet de faisceau partiel (308, 508) stéréoscopique,
l'axe optique (323, 523) du trajet de faisceau (324, 524) mis à disposition au niveau de l'interface optique (322, 522) étant un axe de symétrie qui traverse le deuxième diviseur de faisceau (304, 504), et
le sous-ensemble optique (310, 510) réglable contenant un élément obturateur (320, 520) qui peut être positionné en mobilité linéaire couplé en mobilité avec l'élément miroir (314, 514) qui peut être positionné en mobilité linéaire pour le recouvrement sélectif du premier diviseur de faisceau (302, 502) et la libération du deuxième diviseur de faisceau (304, 504) ou la libération du premier diviseur de faisceau (302, 502) et le recouvrement du deuxième diviseur de faisceau (304, 504).

2. Module de découplage (32, 700) pour le découplage sélectif d'un trajet de faisceau (58, 724) vers une interface optique (46, 722) en position fixe depuis un premier ou un deuxième trajet de faisceau partiel (18, 20, 706, 708) stéréoscopique dans un instrument optique (10),
comprenant un premier diviseur de faisceau (36, 704) disposé dans le premier trajet de faisceau partiel (18, 708) stéréoscopique,
comprenant un deuxième diviseur de faisceau (38, 702) disposé dans le deuxième trajet de faisceau partiel (20, 706) stéréoscopique,
comprenant un sous-ensemble optique (66, 710) réglable qui achemine le trajet de faisceau (58, 724) découplé vers l'interface optique (46, 722) sélectivement depuis le premier diviseur de faisceau (36, 704) ou le deuxième diviseur de faisceau (38, 702),
**caractérisé en ce que**
le sous-ensemble optique (66, 710) réglable contient au moins un élément miroir (67, 714) qui peut être positionné en mobilité linéaire d'une première position dans une deuxième position et inversement par rapport à l'interface optique (46, 722) et qui, dans la première position, est disposé dans le trajet de faisceau (58, 724) acheminé à l'interface optique (46, 722),
dans la première position, l'élément miroir (67, 714) qui peut être positionné en mobilité linéaire recevant le trajet de faisceau (58, 724) acheminé à l'interface optique (46, 722) depuis un élément miroir supplémentaire (70, 716), auquel est acheminé le trajet de faisceau (58, 724) depuis le deuxième diviseur de faisceau (38, 702) pour le découplage hors du deuxième trajet de faisceau partiel (20, 706) stéréoscopique et, dans la deuxième position, libérant le trajet de faisceau (58, 724) acheminé à l'interface optique (46, 722) depuis le deuxième trajet de faisceau partiel (308, 706) stéréoscopique vers l'interface optique (722),
au niveau de l'interface optique (46, 722), l'axe optique (23, 723) du trajet de faisceau (58, 724) acheminé à l'interface optique (46, 722) étant un axe de symétrie qui traverse le premier diviseur de faisceau (36, 704), et
le sous-ensemble optique (66, 710) réglable contenant un élément obturateur (64, 720) qui peut être positionné en mobilité linéaire couplé en mobilité avec l'élément miroir (67, 714) qui peut être positionné en mobilité linéaire pour le recouvrement sélectif du premier diviseur de faisceau (36, 704) et la libération du deuxième diviseur de faisceau (38, 702) ou la libération du premier diviseur de faisceau (36, 704) et le recouvrement du deuxième diviseur de faisceau (38, 702).

3. Module selon la revendication 1 ou 2, **caractérisé en ce que** l'élément miroir supplémentaire (70, 716) est en position fixe par rapport à l'interface optique (46, 322) .

4. Module selon la revendication 1 ou 2, **caractérisé en ce que** l'élément miroir supplémentaire (716) peut être positionné en mobilité linéaire par rapport à l'interface optique (722).

5. Module d'injection (400) pour l'injection sélective d'un trajet de faisceau (424) mis à disposition au niveau d'une interface optique (422) en position fixe dans un premier ou un deuxième trajet de faisceau partiel (406, 408) stéréoscopique dans un instrument optique (10), comprenant un premier diviseur de faisceau (402) disposé dans le premier trajet de faisceau partiel (406) stéréoscopique,
comprenant un deuxième diviseur de faisceau (404) disposé dans le deuxième trajet de faisceau partiel (408) stéréoscopique,
comprenant un sous-ensemble optique (410) réglable qui achemine le trajet de faisceau (424) mis à disposition au niveau de l'interface optique (422) sélectivement au premier diviseur de faisceau (402) ou au deuxième diviseur de faisceau (404),
**caractérisé en ce que**
le sous-ensemble optique (410) réglable contient au moins un élément miroir (414) qui peut être positionné en mobilité linéaire d'une première position dans une deuxième position et inversement par rapport à l'interface optique (422) et qui, dans la première position, est disposé dans le trajet de faisceau (424) mis à disposition au niveau de l'interface optique (422) et achemine le trajet de faisceau (424) mis à disposition au premier diviseur de faisceau (402) pour l'injection dans le premier trajet de faisceau partiel (406) stéréoscopique,
l'élément miroir (414) qui peut être positionné en mobilité linéaire, dans la deuxième position, étant également disposé dans le trajet de faisceau (424) mis à disposition et acheminant le trajet de faisceau au deuxième diviseur de faisceau (404) pour l'injection dans le deuxième trajet de faisceau partiel (408) stéréoscopique et
le sous-ensemble optique (410) réglable contenant un élément obturateur (420) qui peut être positionné en mobilité linéaire couplé en mobilité avec l'élément miroir (414) qui peut être positionné en mobilité linéaire pour le recouvrement sélectif du premier diviseur de faisceau (402) et la libération du deuxième diviseur de faisceau (404) ou la libération du premier diviseur de faisceau (402) et le recouvrement du deuxième diviseur de faisceau (404).

6. Module de découplage (600) pour le découplage sélectif d'un trajet de faisceau (624) vers une interface optique (622) en position fixe depuis un premier ou un deuxième trajet de faisceau partiel (606, 608) stéréoscopique dans un instrument optique (10),
comprenant un premier diviseur de faisceau (602) disposé dans le premier trajet de faisceau partiel (606) stéréoscopique,
comprenant un deuxième diviseur de faisceau (604) disposé dans le deuxième trajet de faisceau partiel (608) stéréoscopique,
comprenant un sous-ensemble optique (610) réglable qui achemine sélectivement à l'interface optique (622) un trajet de faisceau découplé depuis le premier ou le deuxième trajet de faisceau partiel (606, 608) stéréoscopique,
**caractérisé en ce que**
le sous-ensemble optique (610) réglable contient au moins un élément miroir (614) qui peut être positionné en mobilité linéaire d'une première position dans une deuxième position et inversement par rapport à l'interface optique (622) et qui, dans la première position, est disposé dans le trajet de faisceau (624) à découpler et reçoit le trajet de faisceau du premier diviseur de faisceau (602) pour le découplage hors du premier trajet de faisceau partiel (606) stéréoscopique, l'élément miroir (614) qui peut être positionné en mobilité linéaire, dans la deuxième position, étant également disposé dans le trajet de faisceau (624) à découpler et recevant le trajet de faisceau du deuxième diviseur de faisceau (604) pour le découplage hors du deuxième trajet de faisceau partiel (608) stéréoscopique et
le sous-ensemble optique (610) réglable contenant un élément obturateur (620) qui peut être positionné en mobilité linéaire couplé en mobilité avec l'élément miroir (614) qui peut être positionné en mobilité linéaire pour le recouvrement sélectif du premier diviseur de faisceau (602) et la libération du deuxième diviseur de faisceau (604) ou la libération du premier diviseur de faisceau (602) et le recouvrement du deuxième diviseur de faisceau (604).

7. Module selon l'une des revendications 1 à 4, **caractérisé par** un cadre porteur (33) qui accueille le sous-ensemble optique (60), dans lequel l'élément miroir supplémentaire (316) est maintenu au niveau d'un dispositif d'ajustement (72) avec lequel l'élément miroir supplémentaire (316) peut être ajusté pour le réglage de l'axe optique (45) du trajet de faisceau (56') acheminé au premier diviseur de faisceau (40).

8. Module selon l'une des revendications 1 à 7, **caractérisé par** un cadre porteur (33) qui accueille le sous-ensemble optique (410), dans lequel l'élément miroir (414) est maintenu au niveau d'un dispositif d'ajustement (72) avec lequel l'élément miroir (414) peut être ajusté pour le réglage de l'axe optique du trajet de faisceau acheminé au premier diviseur de faisceau (402) et au deuxième diviseur de faisceau (404).

9. Microscope d'opération (10) pour l'observation stéréoscopique d'une zone d'objet (16), comprenant un premier (18) et un deuxième (20) trajet de faisceau partiel stéréoscopique et comprenant un module d'injection (34, 128, 228, 300, 400, 500) configuré selon l'une des revendications 1, 3 à 5, 7 ou 8 et/ou comprenant un module de découplage (32, 134, 234, 600, 700) configuré selon l'une des revendications 2, 3, 4 ou 6 à 8.
